# EUROPEAN PATENT APPLICATION

(11) **EP 3 556 778 A1**
(43) Date of publication of application: **23.10.2019**
(21) Application number: 19176582.5
(22) Date of filing: 30.03.2016
(51) Int. Cl.: C07K 19/00, A61K 38/00, A61P 37/04, A61P 37/06, A61P 37/08, C07K 14/47, C07K 14/74, C12N 15/09

(54) **IMMUNIZING PEPTIDE, METHOD FOR PRODUCING IMMUNIZING PEPTIDE, PHARMACEUTICAL COMPOSITION FOR IMMUNE DISORDERS CONTAINING SAME, AND METHOD FOR TREATING IMMUNE DISORDERS**

(30) Priority: 30.03.2015 JP 2015068043
(62) Divisional of application: 16773062.1
(71) Applicant: Osaka University, Suita-shi, Osaka 565-0871 (JP)
(72) Inventor: ARASE, Hisashi, Osaka, 565-0871 (JP); KISHIDA, Kazuki, Osaka, 565-0871 (JP)
(74) Representative: J A Kemp

(57) **Abstract**

The present invention provides an immunizing peptide that can induce production of an antibody to a complex of the immunizing peptide and an MHC molecule of a living organism. The immunizing peptide according to the present invention includes a T-cell epitope and an antibody-inducing portion of a target protein. The antibody-inducing portion is at least one of the following amino acid residues in an amino acid sequence of the target protein: one or more contiguous amino acid residues immediately upstream of a N-terminal amino acid residue of the T-cell epitope; and one or more contiguous amino acid residues immediately downstream of a C-terminal amino acid residue of the T-cell epitope. When the immunizing peptide is administered to a living organism, the immunizing peptide induces production of an antibody to a complex of the immunizing peptide and an MHC molecule of the living organism.

SEQUENCE LISTING

<110>  Osaka University

<120>  Peptide for immunization, method of producing peptide for
       immunization, pharmaceutical composition for immune diseases
       containing the same, and method of treating immune disease

<130>  TF14085WO

<150>  JP2015-068043
<151>  2015-03-30

<160>  25

<170>  PatentIn version 3.5

<210>  1
<211>  14
<212>  PRT
<213>  Gallus gallus domesticus

<400>  1

Asp Gly Ser Thr Asp Tyr Gly Ile Leu Gln Ile Asn Ser Arg
1               5                   10

<210>  2
<211>  20
<212>  PRT
<213>  Triticum aestivum

<400>  2

Pro Phe Pro Gln Gln Pro Gln Gln Pro Phe Pro Gln Gln Pro Gln Gln
1               5                   10                  15

Ser Phe Pro Gln
            20

<210>  3
<211>  19
<212>  PRT
<213>  Gallus gallus domesticus

<400>  3

Val Thr Tyr Thr Asn Asp Cys Leu Leu Cys Ala Tyr Ser Ile Glu Phe
1               5                   10                  15

Gly Thr Asn

<210>  4
<211>  9
<212>  PRT
<213>  Cryptomeria japonica

<400>  4

Lys Val Thr Val Ala Phe Asn Gln Phe
1               5


<210>  5
<211>  23
<212>  PRT
<213>  Acarina

<400>  5

Ser Ala Tyr Leu Ala His Arg Asn Gln Ser Leu Asp Leu Ala Glu Gln
1               5                   10                  15


Glu Leu Val Asp Cys Ala Ser
            20


<210>  6
<211>  13
<212>  PRT
<213>  Felis silvestris catus

<400>  6

Leu Pro Val Val Leu Glu Asn Ala Arg Ile Leu Lys Asn
1               5                   10


<210>  7
<211>  13
<212>  PRT
<213>  Mus musculus

<400>  7

His Cys Leu Gly Lys Trp Leu Gly His Pro Asp Lys Phe
1               5                   10


<210>  8
<211>  7
<212>  PRT
<213>  Triticum aestivum

<400>  8

Gln Gln Ile Pro Gln Gln Gln
1               5


<210>  9
<211>  40
<212>  PRT
<213>  Gallus gallus domesticus

<400>  9

Ala Glu Val Asp Cys Ser Arg Phe Pro Asn Ala Thr Asp Lys Glu Gly
1               5                   10                  15

Lys Asp Val Leu Ser Ile Glu Phe Gly Thr Asn Ile Ser Lys Val Glu
            20                  25                  30

Gln Gly Ala Ser Val Asp Lys Arg
        35                  40

<210>  10
<211>  20
<212>  PRT
<213>  Cryptomeria japonica

<400>  10

Gly Val Glu Pro Val His Pro Gln Asp Gly Asp Ala Leu Thr Leu Arg
1               5                   10                  15

Thr Ala Thr Asn
            20

<210>  11
<211>  771
<212>  DNA
<213>  Mus musculus

<400>  11
atgccgcgca gcagagctct gattctgggg gtcctcgccc tgaccaccat gctcagcctc      60

tgcggaggtg aagacgacat tgaggccgac cacgtaggct cctatggtat aactgtatat     120

cagtctcctg gagacattgg ccagtacaca tttgaatttg atggtgatga gttgttctat     180

gtggacttgg ataagaagga gactgtctgg atgcttcctg agtttgctca actgagaaga     240

tttgagcccc aaggtggact gcaaaacata gctacaggaa aacacaactt ggaaatcttg     300

actaagaggt caaattccac cccagctacc aatgaggctc ctcaagcgac tgtgttcccc     360

aagtctcctg tgctgctggg tcagcccaac acccttatct gctttgtgga caacatcttc     420

cctcctgtga tcaacatcac atggctcaga aatagcaagt cagtcacaga cggcgtttat     480

gaaaccagct tcttcgtcaa ccgtgactat tccttccaca agctgtctta tctcaccttc     540

atcccttctg acgatgacat ttatgactgc aaggtggagc actggggcct ggaggagccg     600

gttctgaaac actgggaacc tgagattcca gcccccatgt cagagctgac agagactgtg     660

gtgtgtgccc tggggttgtc tgtgggcctt gtgggcatcg tggtgggcac catcttcatc     720

attcaaggcc tgcgatcagg tggcacctcc agacacccag ggcctttatg a              771

<210>  12
<211>  792
<212>  DNA
<213>  Mus musculus

<400>  12
atggctctgc agatccccag cctcctcctc ttggctgctg ttgtggtgct gacggtgctg      60

agcagcccag ggactgaggg cggaaactcc gaaaggcatt tcgtgcacca gttccagccc     120

ttctgctact tcaccaacgg gacgcagcgc atacggcttg tgatcagata catctacaac     180

cgggaggagt acgtgcgctt cgacagcgac gtgggcgagt accgcgcggt gaccgagctg     240

gggcggccag acgccgagta ctggaataag cagtacctgg agcgaacgcg ggccgagctg     300

gacacggtgt gcagacacaa ctacgagaag acggagaccc ccacctccct gcggcggctt     360

gaacagccca gtgtcgtcat ctccctgtcc aggacagagg ccctcaacca ccacaacacg     420

ctggtctgct cagtgacaga tttctaccca gccaagatca aagtgcgctg gttccggaat     480

ggccaggagg agacggtggg ggtctcgtcc acacagctta ttaggaatgg ggactggacc     540

ttccaggtcc tggtcatgct ggagatgacc cctcggcggg gagaggtcta cacctgccat     600

gtggagcatc ccagcctgaa gagtcccatc accgtggagt ggcgggcaca gtccgagtct     660

gcccggagca agatgttgag cggcatcggt ggctgcgtgc ttggggtgat cttcctcggg     720

cttggccttt tcatccgtca caggagtcag aaaggacctc gaggccctcc tccagcaggg     780

ctcctgcagt ga                                                         792

<210>  13
<211>  720
<212>  DNA
<213>  Aequorea coerulescens

<400>  13
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacaa cagccacaac gtctatatca tggccgacaa gcagaagaac     480

ggcatcaagg tgaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actctcggca tggacgagct gtacaagtaa     720

<210>  14
<211>  30

<212>  PRT
<213>  Gallus gallus domesticus

<400>  14

Lys Val Phe Gly Arg Cys Glu Leu Ala Ala Ala Met Lys Arg His Gly
1               5                   10                  15

Leu Asp Asn Tyr Arg Gly Tyr Ser Leu Gly Asn Trp Val Cys
            20                  25                  30

<210>  15
<211>  30
<212>  PRT
<213>  Gallus gallus domesticus

<400>  15

Gln Ala Thr Asn Arg Asn Thr Asp Gly Ser Thr Asp Tyr Gly Ile Leu
1               5                   10                  15

Gln Ile Asn Ser Arg Trp Trp Cys Asn Asp Gly Arg Thr Pro
            20                  25                  30

<210>  16
<211>  14
<212>  PRT
<213>  Gallus gallus domesticus

<400>  16

Asp Gly Ser Thr Asp Tyr Gly Ile Leu Gln Ile Asn Ser Arg
1               5                   10

<210>  17
<211>  30
<212>  PRT
<213>  Gallus gallus domesticus

<400>  17

Ala Lys Phe Glu Ser Asn Phe Asn Thr Gln Ala Thr Asn Arg Asn Thr
1               5                   10                  15

Asp Gly Ser Thr Asp Tyr Gly Ile Leu Gln Ile Asn Ser Arg
            20                  25                  30

<210>  18
<211>  30
<212>  PRT
<213>  Gallus gallus domesticus

<400>  18

Asp Gly Ser Thr Asp Tyr Gly Ile Leu Gln Ile Asn Ser Arg Trp Trp
1               5                   10                  15

Cys Asn Asp Gly Arg Thr Pro Gly Ser Arg Asn Leu Cys Asn
            20                  25                  30

<210>  19
<211>  1158
<212>  DNA
<213>  Artificial Sequence

<220>
<223>  fusion protein

<400>  19
atgctcctgg cactcctccc agtgctgggg atacactttg tcctgagaga tgcccaagct      60

cagtcagtga cacagcccga tgctcgcgtc actgtctctg aaggagcctc tctgcagctg     120

agatgcaagt attcctactc tgggacgcct tatctgttct ggtatgtcca gtacccgcgg     180

caggggctgc agctgctcct caagtactat tccggagacc cagtggttca aggagtgaat     240

ggctttgagg ctgagttcag caagagtaac tcttccttcc acctgcggaa agcctccgtg     300

cactggagcg actcggctgt gtacttctgt gctttaaatt ctggaggaag caatgcaaag     360

ctagccttcg ggaaaggctc taaactctct gttaaatcaa acggcggagg cggatcagga     420

ggaggaggat caggcggagg aggatcagca gtcacccaaa gcccaagaaa caaggtggca     480

gtaacaggag gaaaggtgac attgagctgt aatcagacta ataaccacaa caacatgtac     540

tggtatcggc aggacacggg gcatgggctg aggctgatcc attattcata tggtgctggc     600

agcactgaga aaggagatat ccctgatgga tacaaggcct ccagaccaag ccaagagaac     660

ttctccctca ttctggagtt ggctaccccc tctcagacat cagtgtactt ctgtgccagc     720

ggtgatgaat cggccctacc ggagctctac tttggtgaag gctcaaagct gacagtgctg     780

gaggatctga gaaatgtgac tccacccaag gtctccttgt ttgagccatc aaaagcagag     840

attgcaaaca aacaaaaggc taccctcgtg tgcttggcca ggggcttctt ccctgaccac     900

gtggagctga gctggtgggt gaatggcaag gaggtccaca gtggggtcag cacggaccct     960

caggcctaca aggagagcaa ttatagctac tgcctgagca gccgcctgag ggtctctgct    1020

accttctggc acaatcctcg caaccacttc cgctgccaag tgcagttcca tgggctttca    1080

gaggaggaca agtggccaga gggctcaccc aaacctgtca cacagaacat cagtgcagag    1140

gcctggggcc gagcagac                                                  1158

<210>  20
<211>  13
<212>  PRT
<213>  Mus musculus

<400>  20

His Ser Leu Gly Lys Trp Leu Gly His Pro Asp Lys Phe
1               5                   10

<210>  21
<211>  16
<212>  PRT
<213>  Mus musculus

<400>  21

Arg Val Ser His Ser Leu Gly Lys Trp Leu Gly His Pro Asp Lys Phe
1               5                   10                  15

<210>  22
<211>  771
<212>  DNA
<213>  Mus musculus

<400>  22
atgccgcgca gcagagctct gattctgggg gtcctcgccc tgaccaccat gctcagcctc      60

tgtggaggtg aagacgacat tgaggccgac cacgtaggcg tctatggtac aactgtatat     120

cagtctcctg gagacattgg ccagtacaca catgaatttg atggtgatga gtggttctat     180

gtggacttgg ataagaagga gactatctgg atgcttcctg agtttggcca attgacaagc     240

tttgaccccc aaggtggact gcaaaacata gctacaggaa aatacacctt gggaatcttg     300

actaagaggt caaattccac cccagctacc aatgaggctc ctcaagcgac tgtgttcccc     360

aagtcccctg tgctgctggg tcagcccaac acccttatct gctttgtgga caacatcttc     420

cctcctgtga tcaacatcac atggctcaga aatagcaagt cagtcacaga cggcgtttat     480

gagaccagct tccttgtcaa ccgtgaccat tccttccaca agctgtctta tctcaccttc     540

atcccttctg acgatgatat ttatgactgc aaggtggagc actggggcct ggaggagccg     600

gttctgaaac actgggaacc tgagattcca gcccccatgt cagagctgac agagactgtg     660

gtgtgtgccc tggggttgtc tgtgggcctt gtgggcatcg tggtgggcac catcttcatc     720

attcaaggcc tgcgatcagg tggcacctcc agacacccag ggcctttatg a              771

<210>  23
<211>  792
<212>  DNA
<213>  Mus musculus

<400>  23
atggctctgc agatccccag cctcctcctc tcggctgctg tggtggtgct gatggtgctg      60

agcagcccag ggactgaggg cggagactcc gaaaggcatt tcgtgttcca gttcaagggc     120

gagtgctact tcaccaacgg gacgcagcgc atacgatctg tggacagata catctacaac     180

cgggaggagt acctgcgctt cgacagcgac gtgggcgagt accgcgcggt gaccgagctg     240

gggcggccag accccgagta ctacaataag cagtacctgg agcaaacgcg ggccgagctg     300

gacacggtgt gcagacacaa ctacgagggg gtggagaccc acacctccct gcggcggctt 360

gaacagccca atgtcgtcat ctccctgtcc aggacagagg ccctcaacca ccacaacact 420

ctggtctgct cagtgacaga tttctaccca gccaagatca aagtgcgctg gttccggaat 480

ggccaggagg agacggtggg ggtctcatcc acacagctta ttaggaatgg ggactggacc 540

ttccaggtcc tggtcatgct ggagatgacc cctcggcggg gagaggtcta cacctgccac 600

gtggagcatc cgagcctgaa gagccccatc actgtggagt ggagggcaca gtctgagtct 660

gcccggagca agatgttgag cggcatcggg ggctgcgtgc ttggggtgat ctttctcggg 720

cttggccttt tcatccgtca caggagtcag aaaggacctc gaggccctcc tcctgcaggg 780

ctcctgcagt ga 792

<210> 24
<211> 816
<212> DNA
<213> Mus musculus

<400> 24
atgaagacat cccttcacac tgtattccta ttcttgtggc tgtggatgga ctgggaaagc 60

catggagaga aggtcgagca acaccagtct acactgagtg ttcgagaggg agacagcgct 120

gtcatcaact gcacttacac agatactgcc tcatcatact tcccttggta caagcaagaa 180

gctggaaaga gtctccactt tgtgatagac attcgttcaa atgtggacag aaaacagagc 240

caaagactta cagttttatt ggataagaaa gccaaacgat tctccctgca catcacagcc 300

acacagcctg aagattcagc catctacttc tgtgcagcaa gcccgggagc taacactgga 360

aagctcacgt ttggacacgg caccatcctt agggtccatc caaacatcca gaacccagaa 420

cctgctgtgt accagttaaa agatcctcgg tctcaggaca gcaccctctg cctgttcacc 480

gactttgact cccaaatcaa tgtgccgaaa accatggaat ctggaacgtt catcactgac 540

aaaactgtgc tggacatgaa agctatggat tccaagagca atggggccat tgcctggagc 600

aaccagacaa gcttcacctg ccaagatatc ttcaaagaga ccaacgccac ctaccccagt 660

tcagacgttc cctgtgatgc cacgttgacc gagaaaagct ttgaaacaga tatgaaccta 720

aactttcaaa acctgtcagt tatgggactc cgaatcctcc tgctgaaagt agcgggattt 780

aacctgctca tgacgctgag gctgtggtcc agttga 816

<210> 25
<211> 921
<212> DNA
<213> Mus musculus

<400> 25
atgggtgcaa gactgctctg ctgtgtagca gtttgtctgc ttggggcagg ctcttttgtt 60

gctggagtaa cccagactcc acgatacctg gtcaaagagg aaggacagaa agcacacatg 120

agttgtagtc ctgaaaaagg gcacactgcc ttttactggt atcaacagaa ccagaaacaa 180

gaacttacat ttttgattaa ctttcggaat gaagaaatta tggaacaaac agacttggtc 240

aagaagagat tctcagctaa gtgttcctcg aactcacagt gcatcctgga aatcctatcc 300

tctgaagaag acgactcagc actgtacctc tgtgccagca gccttgtacg agggaactcc 360

gactacacct tcggctcagg gaccaggctt ttggtaatag aggatctgag aaatgtgact 420

ccacccaagg tctccttgtt tgagccatca aaagcagaga ttgcaaacaa acaaaaggct 480

accctcgtgt gcttggccag gggcttcttc cctgaccacg tggagctgag ctggtgggtg 540

aatggcaagg aggtccacag tggggtcagc acggaccctc aggcctacaa ggagagcaat 600

tatagctact gcctgagcag ccgcctgagg gtctctgcta ccttctggca caatcctcga 660

aaccacttcc gctgccaagt gcagttccat gggctttcag aggaggacaa gtggccagag 720

ggctcaccca aacctgtcac acagaacatc agtgcagagg cctggggccg agcagactgt 780

ggaatcactt cagcatccta tcatcagggg gttctgtctg caaccatcct ctatgagatc 840

ctactgggga aggccaccct atatgctgtg ctggtcagtg gcctagtgct gatggccatg 900

gtcaagaaaa aaaattcctg a 921

## Description

### Technical Field

The present invention relates to an immunizing peptide, a method for producing the immunizing peptide, a pharmaceutical composition for an immune disease containing the immunizing peptide, and a method for treating an immune disease.

### Background Art

It is known that an antibody that recognizes a complex of a major histocompatibility complex (MHC) class II molecule and a T-cell epitope presented by the MHC class II molecule inhibits the recognition of the complex by a T-cell receptor specific to the complex (hereinafter, the T-cell receptor also is referred to as "T-cell receptor that recognizes the complex", and the "T-cell receptor" also is referred to as "TCR"). Thus, it can inhibit the onset and the like of an immune disease (Non-Patent Document 1).

In order to acquire the above-described antibody, it is necessary to immunize a living organism not with the T-cell epitope, but with the complex of the T-cell epitope and the MHC class II molecule (also referred to as "T-cell epitope-MHC class II molecule complex" hereinafter) (Non-Patent Documents 2 to 4). However, the MHC class II molecule has a large number of alleles. Besides, the MHC class II molecule expressed in vivo differs from patient to patient. Accordingly, an attempt to acquire an antibody to each T-cell epitope-MHC class II molecule complex for each immune disease for use as a drug requires the following processes: first, in order to obtain cells that produce the antibody, T-cell epitope-MHC class II molecule complexes in a large number of combinations are provided; then, each one of the large number of T-cell epitope-MHC class II molecule complexes is administered to a living organism; and from the living organism, antibody-producing cells that recognize the T-cell epitope-MHC class II molecule complex are collected in the form of monoclonal cells. Accordingly, there is a problem in that the enormous number of combinations of the T-cell epitope-MHC class II molecule complexes makes it difficult to acquire the antibody and use the antibody in treatment.

### Citation List

### Non-Patent Document(s)

[Non-Patent Document 1] Aharoni et al., "Immunomodulation of experimental allergic encephalomyelitis by antibodies to the antigen-Ia complex", Nature, 1991, vol. 351, pp. 147-150
[Non-Patent Document 2] Viville et al., "Mice lacking the MHC class II-associated invariant chain", Cell, 1993, vol. 72, pp. 635-648
[Non-Patent Document 3] Martin et al., "H2-M mutant mice are defective in the peptide loading of class II molecules, antigen presentation, and T cell repertoire selection", Cell, 1996, vol. 84, pp. 543-550
[Non-Patent Document 4] Cosgrove et al., "Mice lacking MHC class II molecules", Cell, 1991, vol. 66, pp. 1051-1066

### Brief Summary of the Invention

### Problem to be Solved by the Invention

With the foregoing in mind, it is an object of the present invention to provide a novel immunizing peptide that can induce production of an antibody to a complex of the immunizing peptide and an MHC molecule of a living organism.

### Means for Solving Problem

In order to achieve the above object, the present invention provides an immunizing peptide including: a T-cell epitope of a target protein; and an antibody-inducing portion of the target protein, wherein the antibody-inducing portion is at least one of the following amino acid residues in an amino acid sequence of the target protein: one or more contiguous amino acid residues immediately upstream of a N-terminal amino acid residue of the T-cell epitope; and one or more contiguous amino acid residues immediately downstream of a C-terminal amino acid residue of the T-cell epitope, and when the immunizing peptide is administered to a living organism, the immunizing peptide induces production of an antibody to a complex of the immunizing peptide and an MHC molecule of the living organism.

The present invention also provides a method for producing an immunizing peptide (also referred to simply as "production method" hereinafter), including the step of: expressing a polynucleotide encoding the immunizing peptide according to the present invention.

The present invention also provides a pharmaceutical composition for an immune disease (also referred to simply as "pharmaceutical composition" hereinafter), containing the immunizing peptide according to the present invention.

The present invention also provides a method for treating an immune disease, including: administering to a living organism at least one of the immunizing peptide according to the present invention and the pharmaceutical composition according to the present invention.

### Effects of the Invention

The immunizing peptide of the present invention can induce production of an antibody to a complex of the immunizing peptide and the MHC molecule of a living organism.

### Brief Description of Drawings

FIG. 1 shows histograms, each showing the amount of binding of an IgG antibody in Example 1A.
FIG. 2 shows histograms, each showing the amount of binding of an IgG antibody in Example 1A.
FIG. 3 shows histograms, each showing the amount of binding of a soluble single-chain TCR in Example 1B.
FIG. 4 is a graph showing the amount of IL-2 produced in Example 1B.
FIG. 5 shows histograms, each showing the amount of binding of an IgG1 antibody in Example 2A.
FIG. 6 shows histograms, each showing the amount of binding of a soluble single-chain TCR in Example 2B.
FIG. 7 shows histograms, each showing the amount of binding of an IgG antibody in Example 3.
FIG. 8 shows histograms, each showing the amount of binding of a soluble single-chain TCR in Example 4.
FIG. 9 shows histograms, each showing the amount of binding of an IgG antibody in Example 5.
FIG. 10 is a graph showing the proportion of GFP-positive cells in CD45-positive cells in Example 6.
FIG. 11 is a graph showing the result of cell proliferation in Example 7.
FIG. 12 is a graph showing the results of clinical score evaluation in Example 7.
FIG. 13 is a graph showing the results of clinical score evaluation in Example 8.

### Mode for Carrying out the Invention

In the immunizing peptide of the present invention, it is preferable that the antibody-inducing portion is at least one of the following amino acid residues in the amino acid sequence of the target protein: three or more contiguous amino acid residues immediately upstream of the N-terminal amino acid residue of the T-cell epitope; and three or more contiguous amino acid residues immediately downstream of the C-terminal amino acid residue of the T-cell epitope.

In the immunizing peptide of the present invention, it is preferable that the produced antibody inhibits binding of a T-cell receptor that recognizes a complex of the T-cell epitope and the MHC molecule of the living organism.

In the immunizing peptide of the present invention, it is preferable that the T-cell epitope is a substituted T-cell epitope configured so that: the substituted T-cell epitope consists of an amino acid sequence obtained by substitution of one or more amino acid residues recognized by a T-cell receptor in the amino acid sequence of the T-cell epitope; and a complex of the substituted amino acid sequence and the MHC molecule is not recognized by the T-cell receptor specific to a complex of the T-cell epitope and the MHC molecule.

The immunizing peptide of the present invention preferably is configured so that it further includes a B-cell epitope of the target protein, and the B-cell epitope is the following substituted B-cell epitope (1) or (2):
(1) a substituted B-cell epitope that consists of an amino acid sequence obtained by deletion, substitution, insertion, and/or addition of one or more amino acid residues in an amino acid sequence of the B-cell epitope and is not recognized by a B-cell receptor specific to the B-cell epitope; and
(2) a substituted B-cell epitope that consists of an amino acid sequence having less than 100% sequence identity to the amino acid sequence of the B-cell epitope and is not recognized by the B-cell receptor specific to the B-cell epitope.

In the immunizing peptide of the present invention, the MHC molecule is, for example, at least one of an MHC class I molecule and an MHC class II molecule.

In the immunizing peptide of the present invention, the target protein is, for example, an antigenic protein involved in an immune disease.

In the immunizing peptide of the present invention, the immune disease is, for example, an allergic disease or an autoimmune disease. The autoimmune disease is, for example, one selected from the group consisting of food allergies, oral allergy syndrome, drug allergies, pollinosis, atopic dermatitis, allergic conjunctivitis, eosinophilic pneumonia, allergic rhinitis, allergic gastroenteritis, contact dermatitis, urticaria, photosensitivity, metal allergies, cat allergies, mite allergies, and asthma. The autoimmune disease is, for example, one selected from the group consisting of rheumatoid arthritis, type I diabetes, systemic lupus erythematosus, Basedow's disease, Hashimoto's disease, psoriasis, pemphigus, bullous pemphigoid, scleroderma, dermatomyositis, interstitial pneumonia, pulmonary alveolar proteinosis, ANCA-associated vasculitis, polymyalgia rheumatica, autoimmune hepatitis, celiac disease, polymyositis, Sjogren's syndrome, IgG4-related disease, vasculitic syndrome, mixed connective tissue disease, Guillain-Barre syndrome, myasthenia gravis, chronic gastritis, chronic atrophic gastritis, primary biliary cirrhosis, ulcerative colitis, Crohn's disease, primary sclerosing cholangitis, autoimmune pancreatitis, aortitis syndrome, Goodpasture's syndrome, rapidly progressive glomerulonephritis, membranous nephropathy, IgA nephropathy, minimal change nephrotic syndrome, megaloblastic anemia, autoimmune hemolytic anemia, autoimmune neutropenia, idiopathic thrombocytopenic purpura, primary hypothyroidism, idiopathic Addison's disease, chronic discoid lupus erythematosus, localized scleroderma, herpes gestationis, linear IgA bullous dermatosis, epidermolysis bullosa acquisita, alopecia areata, vilitigo vulgaris, leukoderma acquisitum centrifugum of Sutton/Sutton nevus, Harada disease, autoimmune optic neuropathy, anti-cardiolipin antibody syndrome, autoimmune inner ear disorder, idiopathic azoospermia, habitual abortion, Behcet's disease, ankylosing spondylitis, multiple sclerosis, and narcolepsy.

It is preferable that the pharmaceutical composition of the present invention further contains an adjuvant.

In the present invention, the term "upstream" means the N-terminal direction in the amino acid sequence of a protein, and the term "downstream" means the C-terminal direction in the amino acid sequence of a protein. In the present invention, the term "treatment" encompasses: prevention of a disease; improvement of the disease; and improvement in prognosis of the disease, for example, and it may mean any of them.

### <Immunizing Peptide>

The immunizing peptide of the present invention is, as described above, an immunizing peptide including: a T-cell epitope of a target protein; and an antibody-inducing portion of the target protein, wherein the antibody-inducing portion is at least one of the following amino acid residues in an amino acid sequence of the target protein: one or more contiguous amino acid residues immediately upstream of a N-terminal amino acid residue of the T-cell epitope (also referred to as "upstream antibody-inducing portion" hereinafter); and one or more contiguous amino acid residues immediately downstream of a C-terminal amino acid residue of the T-cell epitope (also referred to as "downstream antibody-inducing portion" hereinafter), and when the immunizing peptide is administered to a living organism, the immunizing peptide induces production of an antibody to a complex of the immunizing peptide and an MHC molecule of the living organism (also referred to as, "immunizing peptide-MHC molecule complex" hereinafter). The immunizing peptide of the present invention is characterized in that: it includes the T-cell epitope and the antibody-inducing portion of the target protein; the antibody-inducing portion is at least one of the upstream antibody-inducing portion and the downstream antibody-inducing portion in the amino acid sequence of the target protein; and when the immunizing peptide is administered to a living organism, the immunizing peptide induces production of the antibody to the complex of the immunizing peptide and the MHC molecule of the living organism, and other configurations or conditions are not particularly limited. Since the immunizing peptide of the present invention can induce antibody production when administered to a living organism, it also can be referred to as an "antibody production inducing agent", for example.

In order to acquire an antibody to a complex of a T-cell epitope and an MHC class II molecule (also referred to as "T-cell epitope-MHC class II molecule complex" hereinafter), i.e., a complex of a peptide and an MHC class II molecule (also referred to as "peptide-MHC class II molecule complex" hereinafter), it is generally necessary to prepare the peptide-MHC class II molecule complex and perform immunization with the peptide-MHC class II molecule complex. However, the present invention can eliminate the necessity of preparing the immunizing peptide-MHC class II molecule complex when inducing the antibody to the immunizing peptide-MHC class II molecule complex for the reasons to be described below. Specifically, the inventors of the present invention conducted diligent research, and as a result, they found out that, during an immune response, an MHC class II molecule presents a polypeptide (i.e., an immunizing peptide) longer than a T-cell epitope, and also discovered the presence of an antibody to the immunizing peptide-MHC class II molecule complex. Then, the inventors of the present invention verified that production of an antibody to the immunizing peptide-MHC class II molecule complex can be induced by administering the immunizing peptide to a living organism, thereby achieving the present invention. Thus, according to the present invention, it is not necessary to prepare an immunizing peptide-MHC class II molecule complex prior to the induction of antibody production to the immunizing peptide-MHC class II molecule complex as disclosed in the above prior art documents. Therefore, the present invention can induce antibody production easily, for example. The inventors of the present invention also found out that, during an immune response, a T cell having a TCR that recognizes the T-cell epitope-MHC class II molecule complex recognizes the immunizing peptide-MHC class II molecule complex, and that an antibody whose production is induced by the immunizing peptide can inhibit the binding of the TCR to the immunizing peptide-MHC class II molecule complex. The same applies to a complex of the immunizing peptide and an MHC class I molecule (also referred to as "immunizing peptide-MHC class I molecule complex" hereinafter). Therefore, the immunizing peptide of the present invention can induce antibody production to the immunizing peptide-MHC class II molecule complex when administered to a living organism, so that the onset, exacerbation, and the like of a disease caused by a T cell that recognizes the T-cell epitope-MHC molecule complex can be inhibited effectively, for example.

In the present invention, the target protein is not particularly limited, and may be an antigenic protein involved in an immune disease, for example. The immune disease is not particularly limited, and may be an allergic disease, an autoimmune disease, or rejection after organ transplantation, for example. Examples of the allergic disease include food allergies, oral allergy syndrome, drug allergies, pollinosis, atopic dermatitis, allergic conjunctivitis, eosinophilic pneumonia, allergic rhinitis, allergic gastroenteritis, contact dermatitis, urticaria, photosensitivity, metal allergies, cat allergies, mite allergies, and asthma. Examples of the food allergies include egg allergies, peanut allergies, wheat allergies, buckwheat allergies, soy allergies, milk allergies, fish allergies, and fruit allergies. Examples of the pollinosis include Japanese cedar pollinosis, ragweed pollinosis, and white birch pollinosis. Examples of the autoimmune disease include rheumatoid arthritis, type I diabetes, systemic lupus erythematosus (SLE), Basedow's disease, Hashimoto's disease, psoriasis, pemphigus, bullous pemphigoid, scleroderma, dermatomyositis, interstitial pneumonia, pulmonary alveolar proteinosis, antineutrophil cytoplasmic antibody (ANCA)-associated vasculitis, polymyalgia rheumatica, autoimmune hepatitis, celiac disease, polymyositis, Sjogren's syndrome, IgG4-related disease, vasculitic syndrome, mixed connective tissue disease, Guillain-Barre syndrome, myasthenia gravis, chronic gastritis, chronic atrophic gastritis, primary biliary cirrhosis, ulcerative colitis, Crohn's disease, primary sclerosing cholangitis, autoimmune pancreatitis, aortitis syndrome, Goodpasture's syndrome, rapidly progressive glomerulonephritis, membranous nephropathy, IgA nephropathy, minimal change nephrotic syndrome, megaloblastic anemia, autoimmune hemolytic anemia, autoimmune neutropenia, idiopathic thrombocytopenic purpura, primary hypothyroidism, idiopathic Addison's disease, chronic discoid lupus erythematosus, localized scleroderma, herpes gestationis, linear IgA bullous dermatosis, epidermolysis bullosa acquisita, alopecia areata, vilitigo vulgaris, leukoderma acquisitum centrifugum of Sutton/Sutton nevus, Harada disease, autoimmune optic neuropathy, anti-cardiolipin antibody syndrome, autoimmune inner ear disorder, idiopathic azoospermia, habitual abortion, Behcet's disease, ankylosing spondylitis, multiple sclerosis, and narcolepsy. When the target protein is an antigenic protein involved in the allergic disease, the target protein can be referred to as an "allergen", for example. When the target protein is an antigenic protein involved in the autoimmune disease, the target protein can be referred to as an "autoantigenic protein", for example. Specific examples of the target protein include those listed in Tables 1 and 2 below. One type of target protein may be used, or two or more types of target proteins may be used, for example.

**[Table 1]**

| Disease | Target protein (Database No.) | Amino acid sequence of T-cell epitope | MHC class II molecule presenting T-cell epitope |
|---|---|---|---|
| Egg allergy | Hen egg lysozyme (HEL) (1LKS_A) | DGSTDYGILQINSR (SEQ ID NO: 1) | I-A^{k} |
| Wheat allergy | ω-5 gliadin (AAG17702) | PFPQQPQQPFPQQPQQSFPQ (SEQ ID NO: 2) | HLA-DQ2, DQ8 |
| Egg allergy | Ovomucoid (NP_001106132) | VTYTNDCLLCAYSIEFGTN (SEQ ID NO: 3) | HLA-DRB1 |
| Japanese cedar pollinosis | Cry j IA (BAA05542) | KVTVAFNQF (SEQ ID NO: 4) | HLA-DP5 |
| Mite allergy | Der p 1 (AAA28296) | SAYLAHRNQSLDLAEQELVDCAS (SEQ ID NO: 5) | HLA-DR7, DR4 |
| Cat allergy | Fel d 1 (AAC37318) | LPVVLENARILKN (SEQ ID NO: 6) | HLA-DR1 |
| Multiple sclerosis | PLP (myelin proteolipid protein) (NP_001277491.1) | HCLGKWLGHPDKF (SEQ ID NO: 7) | I-A^{s} |

The term "T-cell epitope" as used in the present invention means, for example, the shortest peptide recognized by a specific CD4-positive or CD8-positive T cell when it forms a complex with an MHC class I molecule or MHC class II molecule to be described below. In the present invention, the T-cell epitope may be a T-cell epitope recognized by a CD4-positive T cell, a T-cell epitope recognized by a CD8-positive T cell, or a T-cell epitope recognized by both the CD4-positive T cell and the CD8-positive T cell. The T-cell epitope may be, for example, a known T-cell epitope of the target protein or a T-cell epitope predicted by an algorithm or the like on the basis of: the amino acid sequence of the target protein; and the MHC class I molecule or the MHC class II molecule. Examples of the former include those registered in a known T-cell epitope library "Immune Epitope Database (http://www.iedb.org)". Examples of the latter include those predicted using algorithms in the Immune Epitope Database. Specific examples of the T-cell epitope of the target protein include those listed in Table 1 above. When the target protein includes a plurality of T-cell epitopes, the immunizing peptide may include one of the T-cell epitopes, two or more of the T-cell epitopes, or all the T-cell epitopes, for example.

In the present invention, the T-cell epitope preferably is a substituted T-cell epitope configured so that: the substituted T-cell epitope consists of an amino acid sequence obtained by substitution of one or more amino acid residues recognized by a TCR in the amino acid sequence of the T-cell epitope; and a complex of the substituted amino acid sequence and the MHC molecule is not recognized by the TCR specific to a T-cell epitope-MHC molecule complex. The substituted T-cell epitope is relatively less likely to be recognized or not recognized by a T cell having a TCR that recognizes a complex of the unsubstituted T-cell epitope and an MHC molecule when it forms a complex with the MHC molecule, for example. Thus, when the substituted T-cell epitope is administered to a living organism, it reduces the degree of activation of the T cell having the TCR that recognizes the complex of the unsubstituted T-cell epitope and the MHC molecule and can induce antibody production to the immunizing peptide-MHC molecule complex, for example. Therefore, for example, by administering an immunizing peptide including the substituted T-cell epitope to a patient with an immune disease, it is possible to inhibit the onset, exacerbation, and the like of the immune disease caused by the T cell having the TCR that recognizes the complex of the unsubstituted T-cell epitope and the MHC molecule. When the immunizing peptide includes a plurality of T-cell epitopes, one of the T-cell epitopes may be the substituted T-cell epitope, two or more of the T-cell epitopes may be the substituted T-cell epitopes, or all the T-cell epitopes may be the substituted T-cell epitopes. The living organism will be described below.

The state where a peptide-MHC molecule complex is not recognized by the TCR means, for example, that the responsiveness of a T cell expressing the TCR to the peptide-MHC molecule complex is significantly lower than the responsiveness of the same to a peptide-MHC molecule complex specific to the TCR. The responsiveness can be measured by, for example, the proliferation potency, the IL-2 producibility, the degree of NFAT reporter gene activation, or the like when the T cell expressing the TCR is stimulated with the peptide-MHC molecule complex. The peptide may be an immunizing peptide, a T-cell epitope, or a substituted T-cell epitope.

In the amino acid sequence of the T-cell epitope, an amino acid residue(s) to be substituted is not limited as long as it is at a position recognized by the TCR, and can be set as appropriate depending on the T-cell epitope, for example. The position of the amino acid residue to be substituted may be such that, for example: a substituted T-cell epitope obtained by substituting the amino acid residue at the above-described position in the amino acid sequence of the T-cell epitope forms a complex with the MHC molecule; and recognition by a TCR specific to the T-cell epitope-MHC molecule complex is significantly lowered by the substitution. More specifically, the position of the amino acid residue to be substituted is on a peptide-binding groove of the MHC molecule and is other than the position of the anchor residue, for example. Specific examples of the position of the amino acid residue to be substituted include the positions of the underlined amino acid residues shown in the column entitled "Amino acid sequence of T-cell epitope" in Table 1 above. An amino acid residue after the substitution is different from the amino acid residue before being substituted, for example. Preferably, the amino acid residue after the substitution has a side chain having different properties from the side chain of the amino acid residue before being substituted. Specifically, when the target protein is HEL and the T-cell epitope is a peptide consisting of the amino acid sequence of SEQ ID NO: 1, the positions of the amino acid residues to be substituted are, for example, the 6th tyrosine (Y) (the 53rd tyrosine in the HEL) and the 9th leucine (L) (the 56th leucine in the HEL). The 6th tyrosine (Y) may be alanine (A) after the substitution, for example, and the 9th leucine (L) may be alanine (A) after the substitution, for example. When the target protein is PLP and the T-cell epitope is a peptide consisting of the amino acid sequence of SEQ ID NO: 7, the positions of the amino acid residues to be substituted are, for example, the 3rd leucine (L) (the 141st leucine in the PLP), the 6th tryptophan (W) (the 144th tryptophan in the PLP), and the 9th histidine (H) (the 147th histidine in the PLP). In the amino acid sequence of the immunizing peptide, cysteine (C) may be substituted with serine (S). Specifically, when the target protein is PLP and the T-cell epitope is a peptide consisting of the amino acid sequence of SEQ ID NO: 7, the position of the amino acid residue to be substituted is, for example, the 2nd cysteine (C) (the 140th cysteine in the PLP).

The antibody-inducing portion is at least one of the upstream antibody-inducing portion and the downstream antibody-inducing portion in the amino acid sequence of the target protein. More specifically, the immunizing peptide may include only the upstream antibody-inducing portion, only the downstream antibody-inducing portion, or both the upstream antibody-inducing portion and the downstream antibody-inducing portion, for example. The antibody-inducing portion(s) included in the immunizing peptide can be set as appropriate depending on the T-cell epitope of the target protein, for example. When the target protein is HEL and the T-cell epitope is the peptide consisting of the amino acid sequence of SEQ ID NO: 1, it is preferable that the immunizing peptide includes the downstream antibody-inducing portion. When the target protein is PLP and the T-cell epitope is the peptide consisting of the amino acid sequence of SEQ ID NO: 7, it is preferable that the immunizing peptide includes the upstream antibody-inducing portion.

When the immunizing peptide includes the upstream antibody-inducing portion, the number of the contiguous amino acid residues in the upstream antibody-inducing portion is not limited as long as it is one or more, and may be 2, 3, 4, 5, 10, 15, 20, 25, 30, 35, 40, 45, or more, for example. The upper limit of the number of the contiguous amino acid residues is not particularly limited, and may be as follows, for example: the number of all the amino acid residues upstream of the N-terminal amino acid of the T-cell epitope in the amino acid sequence of the target protein (also referred to as "the total number of N-terminal side amino acid residues" hereinafter), the number obtained by subtracting 1 from the total number of N-terminal side amino acid residues, the number obtained by subtracting 2 from the total number of N-terminal side amino acid residues, the number obtained by subtracting 3 from the total number of N-terminal side amino acid residues, the number obtained by subtracting 4 from the total number of N-terminal side amino acid residues, the number obtained by subtracting 5 from the total number of N-terminal side amino acid residues, the number obtained by subtracting 10 from the total number of N-terminal side amino acid residues, the number obtained by subtracting 15 from the total number of N-terminal side amino acid residues, the number obtained by subtracting 20 from the total number of N-terminal side amino acid residues, the number obtained by subtracting 25 from the total number of N-terminal side amino acid residues, the number obtained by subtracting 30 from the total number of N-terminal side amino acid residues, the number obtained by subtracting 35 from the total number of N-terminal side amino acid residues, the number obtained by subtracting 40 from the total number of N-terminal side amino acid residues, or the number obtained by subtracting 45 from the total number of N-terminal side amino acid residues. As a specific example, when the target protein is HEL and the T-cell epitope is the peptide consisting of the amino acid sequence of SEQ ID NO: 1, the number of the contiguous amino acid residues in the upstream antibody-inducing portion is, 1 to 47, 3 to 23, or 5 to 12, for example. When the target protein is PLP and the T-cell epitope is the peptide consisting of the amino acid sequence of SEQ ID NO: 7, the number of the contiguous amino acid residues in the upstream antibody-inducing portion is 1 to 131, 3 to 66, or 5 to 33, for example. In the present invention, it should be understood that the numerical range regarding the number of amino acid residues discloses all the positive integers falling within that range, for example.

When the immunizing peptide includes the downstream antibody-inducing portion, the number of the contiguous amino acid residues in the downstream antibody-inducing portion is not limited as long as it is one or more, and may be 2, 3, 4, 5, 10, 15, 20, 25, 30, 35, 40, 45, or more, for example. The upper limit of the number of the contiguous amino acid residues is not particularly limited, and may be the number of all the amino acid residues downstream of the C-terminal amino acid of the T-cell epitope in the amino acid sequence of the target protein (also referred to as "the total number of C-terminal side amino acid residues" hereinafter), for example, and preferably is the number obtained by subtracting 1 from the total number of C-terminal side amino acid residues, the number obtained by subtracting 2 from the total number of C-terminal side amino acid residues, the number obtained by subtracting 3 from the total number of C-terminal side amino acid residues, the number obtained by subtracting 4 from the total number of C-terminal side amino acid residues, the number obtained by subtracting 5 from the total number of C-terminal side amino acid residues, the number obtained by subtracting 10 from the total number of C-terminal side amino acid residues, the number obtained by subtracting 15 from the total number of C-terminal side amino acid residues, the number obtained by subtracting 20 from the total number of C-terminal side amino acid residues, the number obtained by subtracting 25 from the total number of C-terminal side amino acid residues, the number obtained by subtracting 30 from the total number of C-terminal side amino acid residues, the number obtained by subtracting 35 from the total number of C-terminal side amino acid residues, the number obtained by subtracting 40 from the total number of C-terminal side amino acid residues, or the number obtained by subtracting 45 from the total number of C-terminal side amino acid residues. As a specific example, when the target protein is HEL and the T-cell epitope is the peptide consisting of the amino acid sequence of SEQ ID NO: 1, the number of the contiguous amino acid residues in the downstream antibody-inducing portion is, 1 to 68, 3 to 34, or 5 to 17, for example. When the target protein is PLP and the T-cell epitope is the peptide consisting of the amino acid sequence of SEQ ID NO: 7, the number of the contiguous amino acid residues in the downstream antibody-inducing portion is 1 to 131, 3 to 66, or 5 to 33, for example.

It is preferable that the immunizing peptide further includes a B-cell epitope of the target protein, and the B-cell epitope is the following substituted B-cell epitope (1) or (2):
(1) a substituted B-cell epitope that consists of an amino acid sequence obtained by deletion, substitution, insertion, and/or addition of one or more amino acid residues in an amino acid sequence of the B-cell epitope and is not recognized by a B-cell receptor specific to the B-cell epitope (also referred to as "BCR" hereinafter); and
(2) a substituted B-cell epitope that consists of an amino acid sequence having less than 100% sequence identity to the amino acid sequence of the B-cell epitope and is not recognized by the B-cell receptor specific to the B-cell epitope.

The substituted B-cell epitope is relatively less likely to be recognized or not recognized by a B cell having a BCR that recognizes the unsubstituted B-cell epitope, for example. Thus, when the substituted B-cell epitope is administered to a living organism, it reduces the degree of activation of the B cell having the BCR that recognizes the unsubstituted B-cell epitope and can induce antibody production to the immunizing peptide-MHC molecule complex, for example. Therefore, for example, by administering an immunizing peptide including the substituted B-cell epitope to a patient with an immune disease, it is possible to inhibit side effects, such as anaphylactic shock, caused by the B cell having the BCR that recognizes the unsubstituted B-cell epitope. The substituted B-cell epitope may be used in combination with the substituted T-cell epitope, for example.

The term "B-cell epitope" as used in the present invention means, for example, the shortest peptide recognized by a specific B cell. The B-cell epitope may be, for example, a known B-cell epitope of the target protein or a B-cell epitope predicted by an algorithm or the like on the basis of the amino acid sequence of the target protein. Examples of the former include those registered in a known B-cell epitope library "Immune Epitope Database (http://www.iedb.org)". Examples of the latter include those predicted using algorithms in the Immune Epitope Database. Specific examples of the B-cell epitope of the target protein include those listed in Table 2 below.

**[Table 2]**

| Disease | Target protein (Database No.) | Amino acid sequence of B-cell epitope |
|---|---|---|
| Wheat allergy | ω-5 gliadin (AAG17702) | QQIPQQQ (SEQ ID NO: 8) |
| Egg allergy | Ovomucoid (NP_001106132) | AEVDCSRFPNATDKEGKDVLSIEFGTNISKVEQGASVDKR (SEQ ID NO: 9) |
| Japanese cedar pollinosis | Cry j IA (BAA05542) | GVEPVHPQDGDALTLRTATN (SEQ ID NO: 10) |

When the target protein includes a plurality of B-cell epitopes, the immunizing peptide may include one of the B-cell epitopes, two or more of the B-cell epitopes, or all the B-cell epitopes, for example. When the immunizing peptide includes a plurality of B-cell epitopes, one of the B-cell epitopes may be the substituted B-cell epitope, two or more of the B-cell epitopes may be the substituted B-cell epitopes, or all the B-cell epitopes may be the substituted B-cell epitopes. The immunizing peptide may include the whole or part of the amino acid sequence of each B-cell epitope, for example.

The "one or more" amino acid residues in the above item (1) are not limited as long as the substituted B-cell epitope (1) is not recognized by the BCR specific to the B-cell epitope, for example. The "one or more" amino acid residues in the item (1) is, for example, 1, 2, 1 to 3, 1 to 5, 1 to 9, 1 to 10, 1 to 20, 1 to 30, 1 to 40, 1 to 45, or 1 to 50 amino acid residues in the amino acid sequence of the B-cell epitope. The upper limit of "one or more" is 100, 90, or 80, for example.

The "sequence identity" in the above item (2) is not limited as long as the substituted B-cell epitope (2) is not recognized by the BCR specific to the B-cell epitope, for example. It is only required that the sequence identity of the substituted B-cell epitope (2) is less than 100% to the amino acid sequence of the B-cell epitope, and is, for example, 99% or less, 98% or less, 97% or less, 96% or less, 95% or less, 90% or less, 85% or less, 80% or less, 70% or less, 60% or less, 50% or less, or 40% or less. The lower limit of the sequence identity is not particularly limited, and is, for example, 0%, 5%, 10%, 20%, or 30%. The range of the sequence identity is, for example, not less than 0% and less than 100%, not less than 5% and not more than 95%, or not less than 10% and not more than 90%.

The state where the B-cell epitope is not recognized by the BCR means, for example, that the binding ability of a B cell expressing the BCR to the B-cell epitope is significantly lower than the binding ability of the same to a B-cell epitope specific to the BCR, for example. The binding ability can be measured by, for example, an ELISA method, flow cytometry, or an intermolecular interaction analysis.

The immunizing peptide induces production of an antibody to the immunizing peptide-MHC molecule complex when administered to a living organism. The antibody may be, for example, an antibody to the immunizing peptide-MHC class I molecule complex, an antibody to the immunizing peptide-MHC class II molecule complex, or an antibody to both of them. The immunizing peptide may be one type of immunizing peptide, or may be a mixture of two or more types of immunizing peptides, for example. The living organism (also referred to as "administration subject" hereinafter) is not particularly limited, and examples thereof include humans and non-human animals excluding humans. Examples of the non-human animals include mice, rats, dogs, monkeys, rabbits, sheep, horses, guinea pigs, and cats.

The dose of the immunizing peptide is not particularly limited, and can be set as appropriate depending on the type, symptom, and age of the administration subject, and the administration method, for example. As a specific example, when the immunizing peptide is administered to a human, the dose of the immunizing peptide per administration is 0.8 to 30 mg or 10 to 30 mg, for example. The number of administrations of the immunizing peptide is not particularly limited, and is 1 to 3 times, for example.

The administration method of the immunizing peptide is not particularly limited, and may be, for example, intravenous injection, intramuscular injection, subcutaneous administration, intradermal administration, transdermal administration, rectal administration, intraperitoneal administration, local administration, transnasal administration, or sublingual administration.

The immunizing peptide may include other components. The other components are not particularly limited, and may be, for example, an adjuvant, a preservative, an antioxidant, or a chelating agent. Examples of the adjuvant include known adjuvants such as aluminium hydroxide, aluminum phosphate, aluminum chloride, a complete Freund's adjuvant, an incomplete Freund's adjuvant, and a Toll-like receptor stimulating agent containing CpG oligonucleotide. Examples of the preservative include thimerosal and 2-phenoxyethanol. Examples of the chelating agent include ethylenediaminetetraacetic acid and glycol ether diaminetetraacetic acid.

The MHC molecule is at least one of an MHC class I molecule and an MHC class II molecule, for example. When the immunizing peptide includes a T-cell epitope recognized by a CD8-positive T cell, the MHC molecule preferably is an MHC class I molecule. When the immunizing peptide includes a T-cell epitope recognized by a CD4-positive T cell, the MHC molecule preferably is an MHC class II molecule.

The MHC class II molecule is a complex of an α-chain and a β-chain, for example. The types of the α-chain and the β-chain are not particularly limited, and the haplotypes of the genes encoding the α-chain and the β-chain are not particularly limited.

When the MHC class II molecule is derived from a human, the α-chain of the MHC class II molecule is, for example, an α-chain of the MHC class II molecule encoded by an HLA-DPA gene locus, an HLA-DQA gene locus, or an HLA-DRA gene locus, and the β-chain of the MHC class II molecule is, for example, a β-chain of the MHC class II molecule encoded by an HLA-DPB gene locus, an HLA-DQB gene locus, or an HLA-DRB gene locus. The haplotypes of the α-chain and β-chain of the MHC class II molecule at the respective gene loci are not particularly limited. The MHC class II molecule is, for example, a molecule including either one of the α-chain and the β-chain, and preferably is a molecule including both the α-chain and the β-chain.

The MHC class II molecule preferably is HLA-DR, HLA-DP, or HLA-DQ, for example. In particular, examples of the MHC class II molecule include those listed in the column entitled "MHC class II molecules presenting T-cell epitope" in Table 1 above.

Examples of the HLA-DR include HLA-DR1, HLA-DR2, HLA-DR3, HLA-DR4, HLA-DR5, HLA-DR6, HLA-DR7, HLA-DR8, HLA-DR9, HLA-DR10, HLA-DR11, HLA-DR12, HLA-DR13, HLA-DR14, HLA-DR15, HLA-DR52, and HLA-DR53. The HLA-DR may be, for example, a molecule including: HLA-DRA such as HLA-DRA1 as the α-chain; and HLA-DRB such as HLA-DRB1, HLA-DRB3, HLA-DRB4, or HLA-DRB5 as the β-chain. Specific examples of the α-chain include alleles such as HLA-DRA1*01, and specific examples of the β-chain include alleles such as HLA-DRB1*01, HLA-DRB1*03, HLA-DRB1*04, HLA-DRB1*07, HLA-DRB1*08, HLA-DRB1*09, HLA-DRB1*10, HLA-DRB1*11, HLA-DRB1*12, HLA-DRB1*13, HLA-DRB1*14, HLA-DRB1*15, HLA-DRB1*16, HLA-DRB3*01, HLA-DRB4*01, and HLA-DRB5*01.

Examples of the HLA-DQ include HLA-DQ1, HLA-DQ2, HLA-DQ3, HLA-DQ4, HLA-DQ5, HLA-DQ6, HLA-DQ7, and HLA-DQ8. The HLA-DQ may be, for example, a molecule including: HLA-DQA such as HLA-DQA1 as the α-chain; and HLA-DQB such as HLA-DQB1 as the β-chain. Specific examples of the α-chain include alleles such as HLA-DQA1*01, HLA-DQA1*02, HLA-DQA1*03, HLA-DQA1*04, HLA-DQA1*05, and HLA-DQA1*06, and specific examples of the β-chain include alleles such as HLA-DQB1*02, HLA-DQB1*03, HLA-DQB1*04, HLA-DQB1*05, and HLA-DQB1*06.

Examples of the HLA-DP include HLA-DP1, HLA-DP2, HLA-DP3, HLA-DP4, and HLA-DP5. The HLA-DP may be, for example, a molecule including: HLA-DPA such as HLA-DPA1 as the α-chain; and HLA-DPB such as HLA-DPB1 as the β-chain. Specific examples of the α-chain include alleles such as HLA-DPA1*01, HLA-DPA1*02, HLA-DPA1*03, and HLA-DPA1*04, and specific examples of the β-chain include alleles such as HLA-DPB1*02, HLA-DPB1*04, HLA-DPB1*05, and HLA-DPB1*09.

The MHC class I molecule is a complex of an α-chain and a β-microglobulin, for example. The type of the α-chain is not particularly limited, and the haplotype of the gene encoding the α-chain is not particularly limited.

When the MHC class I molecule is derived from a human, the α-chain of the MHC class I molecule is, for example, an α-chain of an MHC class I molecule encoded by an HLA-A gene locus, an HLA-B gene locus, or an HLA-C gene locus. The haplotypes of the α-chain of the MHC class I molecule at the respective gene loci are not particularly limited. The MHC class I molecule is, for example, a molecule including either one of the α-chain and β-microglobulin, and preferably is a molecule including both the α-chain and the β-microglobulin.

The MHC class I molecule preferably is HLA-A, HLA-B, or HLA-C, for example.

Examples of the HLA-A include alleles such as HLA-A*02 : 03, HLA-A*02:06, HLA-A*02:07, HLA-A*02:10, HLA-A*02:18, HLA-A*11:01, HLA-A*11:02, HLA-A*24:02, HLA-A*24:04, HLA-A*24:08, HLA-A*24:20, HLA-A*26:01, HLA-A*26:02, HLA-A*26:03, HLA-A*26:04, HLA-A*26:05, and HLA-A*26:06.

Examples of the HLA-B include alleles such as HLA-B*13:01, HLA-B*13:02, HLA-B*15:01, HLA-B*15:02, HLA-B*15:07, HLA-B*15:11, HLA-B*15:18, HLA-B*15:27, HLA-B*39:01, HLA-B*39:02, HLA-B*39:04, HLA-B*40:02, HLA-B*40:03, and HLA-B*40:06.

Examples of the HLA-C include alleles such as HLA-Cw*01:02, HLA-Cw*02:02, HLA-Cw*03:02, HLA-Cw*03:03, HLA-Cw*03:07, HLA-Cw*04:01, HLA-Cw*05:01, and HLA-Cw*08:01.

It is only required that the produced antibody binds to the immunizing peptide-MHC molecule complex, and the antibody may bind to other proteins and the like. The produced antibody may bind to a complex of a shortened peptide resulting from shortening of the administered immunizing peptide and the MHC molecule, for example. The shortened immunizing peptide is, for example, a peptide longer than the T-cell epitope and obtained by deletion of one or more amino acid residues contiguous from an amino acid residue at the exposed end of the antibody-inducing portion in the administered immunizing peptide.

The combination of the immunizing peptide and the MHC molecule in the immunizing peptide-MHC molecule complex is not particularly limited, and may be the combination of the administered immunizing peptide and an MHC molecule expressed in a living organism to which the immunizing peptide has been administered, for example. Specific examples of the combination include the combinations of the immunizing peptide including the T-cell epitope and the MHC molecule shown in Table 1 above. The combination may be one of the combinations shown in Table 1, or two or more of the combinations shown in Table 1, for example.

Preferably, the produced antibody inhibits the binding of a TCR that recognizes the T-cell epitope-MHC molecule complex. More preferably, the produced antibody inhibits the binding of the TCR that recognizes the T-cell epitope-MHC molecule complex to the immunizing peptide-MHC molecule complex. The inhibition may be inhibition of the binding of a TCR that recognizes the T-cell epitope-MHC class I molecule complex, or inhibition of the binding of a TCR that recognizes the T-cell epitope-MHC class II molecule complex, for example. In the former case, the produced antibody preferably inhibits the binding of the TCR to the immunizing peptide-MHC class I molecule complex. In the latter case, the produced antibody preferably inhibits the binding of the TCR to the immunizing peptide-MHC class II molecule complex. The inhibition may be inhibition of both the former and the latter. As described above, the TCR that recognizes the T-cell epitope-MHC molecule complex also recognizes the immunizing peptide-MHC molecule complex, for example. Thus, it also can be said that the produced antibody inhibits the binding of the TCR that recognizes the immunizing peptide-MHC molecule complex, for example. The produced antibody can further reduce the degree of activation of the T cell having the TCR in the administration subject by inhibiting the binding of the TCR that recognizes the T-cell epitope-MHC molecule complex, for example. Therefore, for example, when the immunizing peptide is administered to a living organism, the onset, exacerbation, and the like of the immune disease caused by a T cell that recognizes the T-cell epitope-MHC molecule complex can be inhibited more effectively.

The method for producing the immunizing peptide is not particularly limited. For example, the immunizing peptide may be produced by chemical synthesis, degradation of the target protein, or synthesis using recombinant DNA technology. In the case of the chemical synthesis, the immunizing peptide can be produced by a known organic synthesis method using a protecting group such as a benzyloxycarbonyl group (Cbz), a tert-butoxycarbonyl group (Boc), or a fluorenylmethoxycarbonyl group (Fmoc), for example. In the case of the degradation of the target protein, the immunizing peptide can be produced by degrading the target protein with a known enzyme that performs proteolysis, such as protease or peptidase, for example. Conditions for degrading the target protein can be set as appropriate depending on the type of the target protein, the substrate specificity of the enzyme, and the like, for example. When the recombinant DNA technology is used, the immunizing peptide can be produced in the following manner, for example. First, an expression vector containing a polynucleotide encoding the immunizing peptide is prepared. Then, an expression system for the immunizing peptide is provided, and the immunizing peptide expressed therein is obtained. The expression system can be prepared by introducing the expression vector to a host, for example. The host may be a known host such as an animal cell, a plant cell, an insect cell, or a bacterium, for example. When the recombinant DNA technology is used, the immunizing peptide also may be produced using the polynucleotide encoding the immunizing peptide and a known cell-free translation system, for example. The immunizing peptide can be produced by isolating the immunizing peptide translated from the polynucleotide using the cell-free translation system.

### <Polynucleotide>

The polynucleotide of the present invention is a polynucleotide encoding the immunizing peptide of the present invention. The polynucleotide of the present invention is characterized in that it is a polynucleotide encoding the immunizing peptide of the present invention, and other configurations or conditions are not particularly limited. The polynucleotide of the present invention is useful in synthesis of the immunizing peptide of the present invention by a genetic engineering procedure, for example. The above description regarding the immunizing peptide of the present invention also applies to the polynucleotide of the present invention, for example.

The polynucleotide of the present invention can be designed by replacing the respective amino acid residues in the amino acid sequence of the immunizing peptide with corresponding codons, for example.

The polynucleotide of the present invention may be, for example, a polynucleotide encoding a polypeptide that consists of an amino acid sequence obtained by deletion, substitution, insertion, and/or addition of one to several amino acid residues in the amino acid sequence of the immunizing peptide and induces, when administered to a living organism, antibody production to a complex of the administered polypeptide and an MHC molecule of the living organism, or may be a polynucleotide encoding a polypeptide that consists of an amino acid sequence having a sequence identity of at least 80% to the amino acid sequence of the immunizing peptide and induces, when administered to a living organism, antibody production to a complex of the administered polypeptide and an MHC molecule of the living organism. The "one to several" as used in connection with the above amino acid sequence means, for example, 1 to 20, 1 to 10, 1 to 9, 1 to 5, 1 to 3, or 1 or 2 amino acid residues in the amino acid sequence of the immunizing peptide, for example. The "sequence identity" as used in connection with the above amino acid sequence is, for example, at least 80%, at least 85%, at least 90%, at least 95%, at least 96%, at least 97%, at least 98%, or at least 99% to the amino acid sequence of the immunizing peptide, for example.

In the present invention, the polynucleotide can be synthesized by a genetic engineering procedure or an organic synthesis procedure, for example, and also can be referred to as synthetic DNA (such as cDNA) or synthetic RNA.

### <Expression vector for immunizing peptide>

The expression vector for the immunizing peptide of the present invention (also referred to simply as "expression vector" hereinafter) includes the polynucleotide of the present invention. The expression vector of the present invention is characterized in that it includes the polynucleotide of the present invention, and other configurations or conditions are not particularly limited. The above descriptions regarding the immunizing peptide and polynucleotide of the present invention etc. also apply to the expression vector of the present invention, for example.

The configuration of the expression vector of the present invention is by no means limited as long as the expression vector includes the polynucleotide of the present invention in such a manner that an immunizing peptide encoded by the polynucleotide of the present invention can be expressed, for example.

The expression vector of the present invention can be produced by inserting the polynucleotide of the present invention to a vector forming a main structure (also referred to as "basic vector" hereinafter), for example. The type of the vector is not particularly limited, and can be determined as appropriate depending on the type of the host, for example. Examples of the vector include viral vectors and non-viral vectors. When the vector is introduced by transforming a host using a heat shock method, the vector may be a binary vector, for example. Examples of the vector include a pETDuet-1 vector, a pQE-80L vector, and a pUCP26Km vector. When a bacterium such as *Escherichia coli* is transformed, the vector may be a pETDuet-1 vector (Novagen) or a pQE-80L vector (QIAGEN), for example.

The expression vector of the present invention preferably includes a regulatory sequence that regulates the expression of the polynucleotide of the present invention and the expression of the immunizing peptide of the present invention encoded by the polynucleotide of the present invention, for example. The regulatory sequence may be a promoter, a terminator, an enhancer, a polyadenylation signal sequence, or a replication origin sequence (ori), for example. In the expression vector of the present invention, the arrangement of the regulatory sequence is not particularly limited. In the expression vector of the present invention, it is only required that the regulatory sequence is arranged so that, for example, it can functionally regulate the expression of the polynucleotide of the present invention and the expression of the immunizing peptide of the present invention encoded by the polynucleotide, and the regulatory sequence can be arranged on the basis of a known method. As the regulatory sequence, a sequence originally included in the basic vector may be used, for example. Alternatively, the regulatory sequence further may be inserted into the basic vector, or the regulatory sequence originally included in the basic vector may be replaced with another regulatory sequence.

The expression vector of the present invention further may include a sequence encoding a selection marker, a protein tag, or the like, for example. The selection marker may be a drug-resistant marker, a fluorescent protein marker, an enzyme marker, or a cell surface receptor marker, for example. The tag may be a known protein tag such as a histidine tag, for example.

### <Method for producing immunizing peptide>

As described above, the method for producing the immunizing peptide of the present invention includes the step of: expressing a polynucleotide encoding the immunizing peptide of the present invention. The production method of the present invention is characterized in that it includes the step of expressing a polynucleotide encoding the immunizing peptide of the present invention, and other steps or conditions are not particularly limited. The above descriptions regarding the immunizing peptide, polynucleotide, and expression vector of the present invention etc. also apply to the production method of the present invention.

The polynucleotide of the present invention may be expressed by using the expression vector of the present invention, for example.

The method for expressing the polynucleotide of the present invention is not particularly limited, and a known method may be employed. For example, a host may be used, or a cell-free protein synthesis system may be used.

In the former case, for example, it is preferable to use the host to which the polynucleotide of the present invention has been introduced, and to express the polynucleotide of the present invention in the host by culturing the host. For example, by introducing the polynucleotide of the present invention to the host as described above, a transformant for synthesizing the immunizing peptide of the present invention can be produced, and the immunizing peptide can be synthesized by culturing the transformant.

Examples of the host include: non-human hosts such as microorganisms, animal cells, insect cells, and cultured cells thereof; and isolated human cells and cultured cells thereof. Examples of the microorganisms include prokaryotes and eukaryotes. Examples of the prokaryotes include bacteria belonging to the genus *Escherichia*, such as *Escherichia coli* and bacteria belonging to the genus *Pseudomonas*, such as *Pseudomonas putida.* Examples of the eukaryotes include yeasts such as *Saccharomyces cerevisiae.* Examples of the animal cells include COS cells and CHO cells. Examples of the insect cells include Sf9 and Sf21.

The method for culturing the host is not particularly limited, and can be set as appropriate depending on the type of the host. The medium used for culturing the host is not particularly limited, and can be determined as appropriate depending on the type of the host.

The method for introducing the polynucleotide of the present invention to the host is not particularly limited, and a known method may be used. The polynucleotide of the present invention may be introduced using the expression vector of the present invention, for example. The method for introducing the polynucleotide can be set as appropriate depending on the type of the host, for example. The method for introducing the polynucleotide may be introduction using a gene gun such as a particle gun, a calcium phosphate method, a polyethylene glycol method, a lipofection method using a liposome, an electroporation method, a nucleic acid introduction using ultrasonic waves, a DEAE-dextran method, a direct injection using a minute glass tube or the like, a hydrodynamic method, a cationic liposome method, a method using an adjuvant for helping introduction, and an agrobacterium-mediated method. Examples of the liposome include Lipofectamine® and cationic liposomes. Examples of the adjuvant for helping introduction include atelocollagen, nano-particles, and polymers. When the host is a microorganism, a method mediated by, for example, *E*. *coli* or *Ps. putida* is particularly preferable.

In the latter case, it is preferable to express the polynucleotide of the present invention in a cell-free protein synthesis system. In this case, the polynucleotide of the present invention may be expressed using the expression vector of the present invention. The cell-free protein synthesis system can be constructed by a known method using, for example, a cell extract, a buffer containing respective components, and an expression vector to which a polynucleotide encoding an immunizing peptide to be obtained has been introduced, and a commercially available reagent kit can be used, for example.

The production method of the present invention may include, instead of the expression step, the step of synthesizing the amino acid sequence of the immunizing peptide of the present invention or the step of generating the immunizing peptide of the present invention by degrading the target protein, for example. The above description regarding the immunizing peptide of the present invention, for example, also applies to the synthesis step and the generation step.

The production method of the present invention may further include the step of purifying the immunizing peptide, for example. The method for purifying the immunizing peptide is not particularly limited, and may be a known purification method such as gel filtration or HPLC, for example.

### <Pharmaceutical composition for immune disease>

As described above, the pharmaceutical composition for an immune disease according to the present invention contains the immunizing peptide of the present invention. The pharmaceutical composition of the present invention is characterized in that it contains the immunizing peptide of the present invention, and other configurations or conditions are not particularly limited. The pharmaceutical composition of the present invention can treat the immune disease, for example. The pharmaceutical composition of the present invention can induce antibody production to a immunizing peptide-MHC molecule complex when administered to a living organism. Thus, the pharmaceutical composition of the present invention also can be referred to as "vaccine", for example. The above description regarding the immunizing peptide of the present invention, for example, also applies to the pharmaceutical composition of the present invention.

The pharmaceutical composition of the present invention may contain at least one of the polynucleotide of the present invention and the expression vector of the present invention, instead of or in addition to the immunizing peptide. In this case, the pharmaceutical composition of the present invention also can be referred to as "nucleic acid vaccine", for example.

The pharmaceutical composition of the present invention preferably is used for an immune disease in which the target protein of the immunizing peptide is involved, for example. Examples of the combination of the target protein and the immune disease include those listed in Tables 1 and 2 above. As a specific example, when the target protein is HEL, the immune disease may be egg allergy.

### <Method for designing immunizing peptide>

A method for designing the immunizing peptide of the present invention includes the design step of designing, as an immunizing peptide, a peptide including: a T-cell epitope of a target protein; and an antibody-inducing portion of the target protein, wherein the antibody-inducing portion is at least one of the following amino acid residues in an amino acid sequence of the target protein: one or more contiguous amino acid residues immediately upstream of a N-terminal amino acid residue of the T-cell epitope; and one or more contiguous amino acid residues immediately downstream of a C-terminal amino acid residue of the T-cell epitope. According to the design method of the present invention, the immunizing peptide of the present invention can be designed, for example.

The design method of the present invention is characterized in that it includes the design step of designing, as an immunizing peptide, a peptide including: a T-cell epitope of a target protein; and an antibody-inducing portion of the target protein, wherein the antibody-inducing portion is at least one of the following amino acid residues in an amino acid sequence of the target protein: one or more contiguous amino acid residues immediately upstream of a N-terminal amino acid residue of the T-cell epitope; and one or more contiguous amino acid residues immediately downstream of a C-terminal amino acid residue of the T-cell epitope. Other steps or conditions are not particularly limited. The above description regarding the immunizing peptide of the present invention, for example, also applies to the design method of the present invention.

### <Method for treating immune disease>

As described above, the method for treating an immune disease according to the present invention includes the step of: administering to a living organism at least one of the immunizing peptide according to the present invention and the pharmaceutical composition for an immune disease according to the present invention. The treatment method of the present invention is characterized in that it includes administering to a living organism at least one of the immunizing peptide according to the present invention and the pharmaceutical composition according to the present invention, and other steps or conditions are not particularly limited. The treatment method of the present invention can treat the immune disease, for example. The above descriptions regarding the immunizing peptide of the present invention etc. also apply to the method for treating an immune disease according to the present invention, for example.

In the treatment method of the present invention, at least one of the polynucleotide of the present invention and the expression vector of the present invention may be administered, instead of or in addition to the immunizing peptide and the pharmaceutical composition.

### <Method for inducing antibody>

A method for inducing an antibody according to the present invention includes an antibody induction step of inducing production of an antibody to a complex of an immunizing peptide and an MHC molecule of a living organism by administering to the living organism at least one of the immunizing peptide of the present invention and the pharmaceutical composition of the present invention. The antibody induction method of the present invention is characterized in that it includes administering to the living organism at least one of the immunizing peptide of the present invention and the pharmaceutical composition of the present invention, and other steps or conditions are not particularly limited. According to the antibody induction method of the present invention, antibody production to an immunizing peptide-MHC molecule complex can be induced easily in the living organism, for example. The above descriptions regarding the immunizing peptide and treatment method of the present invention etc. also apply to the antibody induction method of the present invention, for example.

### <Use of immunizing peptide and pharmaceutical composition for immune disease>

The present invention relates to the use of an immunizing peptide for treatment of an immune disease. The present invention also relates to the use of the immunizing peptide for production of a pharmaceutical for an immune disease. The present invention also relates to the use of the pharmaceutical composition for treatment of an immune disease. Also, the present invention relates to the use of the pharmaceutical composition for an immune disease for production of a pharmaceutical for the immune disease. The above descriptions regarding the immunizing peptide of the present invention etc. also apply to the use of the immunizing peptide and pharmaceutical composition according to the present invention, for example.

The present invention will be described in detail below with reference to examples and the like. It is to be noted, however, that the present invention is by no means limited thereto.

### Examples

### [Example 1]

The present example examined whether immunization with an HEL protein induces production of an antibody to a complex of a peptide longer than a T-cell epitope in the HEL protein and an MHC class II molecule of a living organism and whether the antibody inhibits the binding of a TCR that recognizes a T-cell epitope-MHC class II molecule complex.

### (Example 1A)

The present example examined whether immunization with an HEL protein induces production of an antibody to a complex of a peptide longer than a T-cell epitope in the HEL protein and an MHC class II molecule of a living organism.

### (1) Preparation of HEL peptide-presenting cells

From mouse spleen cDNA, polynucleotides encoding the α-chain and the β-chain of a mouse MHC class II molecule (I-A^{k}) shown below were cloned into pME18S vectors, respectively. Thus, a mouse MHC class II molecule (I-A^{k}) α-chain expression vector (I-A^{k} α expression vector) and a mouse MHC class II molecule (I-A^{k}) β-chain expression vector (I-A^{k} β expression vector) were produced. As a GFP expression vector, pMX-GFP (obtained from the Division of Cellular Therapy in the Advanced Clinical Research Center of the Institute of Medical Science, the University of Tokyo) was used.
α-chain of mouse MHC class II molecule (I-A^{k}) (SEQ ID NO: 11) 
ATGCCGCGCAGCAGAGCTCTGATTCTGGGGGTCCTCGCCCTGACCACCA
TGCTCAGCCTCTGCGGAGGTGAAGACGACATTGAGGCCGACCACGTAGG
CTCCTATGGTATAACTGTATATCAGTCTCCTGGAGACATTGGCCAGTACA
CATTTGAATTTGATGGTGATGAGTTGTTCTATGTGGACTTGGATAAGAAG
GAGACTGTCTGGATGCTTCCTGAGTTTGCTCAACTGAGAAGATTTGAGC
CCCAAGGTGGACTGCAAAACATAGCTACAGGAAAACACAACTTGGAAAT
CTTGACTAAGAGGTCAAATTCCACCCCAGCTACCAATGAGGCTCCTCAA
GCGACTGTGTTCCCCAAGTCTCCTGTGCTGCTGGGTCAGCCCAACACCC
TTATCTGCTTTGTGGACAACATCTTCCCTCCTGTGATCAACATCACATGG
CTCAGAAATAGCAAGTCAGTCACAGACGGCGTTTATGAAACCAGCTTCTT
CGTCAACCGTGACTATTCCTTCCACAAGCTGTCTTATCTCACCTTCATCC
CTTCTGACGATGACATTTATGACTGCAAGGTGGAGCACTGGGGCCTGGA
GGAGCCGGTTCTGAAACACTGGGAACCTGAGATTCCAGCCCCCATGTCA
GAGCTGACAGAGACTGTGGTGTGTGCCCTGGGGTTGTCTGTGGGCCTTG
TGGGCATCGTGGTGGGCACCATCTTCATCATTCAAGGCCTGCGATCAGG
TGGCACCTCCAGACACCCAGGGCCTTTATGA
β-chain of mouse MHC class II molecule (I-A^{k}) (SEQ ID NO: 12) 
ATGGCTCTGCAGATCCCCAGCCTCCTCTTGGCTGCTGTTGTGGTGC
TGACGGTGCTGAGCAGCCCAGGGACTGAGGGCGGAAACTCCGAAAGGC
ATTTCGTGCACCAGTTCCAGCCCTTCTGCTACTTCACCAACGGGACGCA
GCGCATACGGCTTGTGATCAGATACATCTACAACCGGGAGGAGTACGTG
CGCTTCGACAGCGACGTGGGCGAGTACCGCGCGGTGACCGAGCTGGGG
 
CGGCCAGACGCCGAGTACTGGAATAAGCAGTACCTGGAGCGAACGCGG
GCCGAGCTGGACACGGTGTGCAGACACAACTACGAGAAGACGGAGACC
CCCACCTCCCTGCGGCGGCTTGAACAGCCCAGTGTCGTCATCTCCCTGT
CCAGGACAGAGGCCCTCAACCACCACAACACGCTGGTCTGCTCAGTGAC
AGATTTCTACCCAGCCAAGATCAAAGTGCGCTGGTTCCGGAATGGCCAG
GAGGAGACGGTGGGGGTCTCGTCCACACAGCTTATTAGGAATGGGACT
GGACCTTCCAGGTCCTGGTCATGCTGGAGATGACCCCTCGGCGGGAGA
GGTCTACACCTGCCATGTGGAGCATCCCAGCCTGAAGAGTCCCATCACC
GTGGAGTGGCGGGCACAGTCCGAGTCTGCCCGGAGCAAGATGTTGAGC
GGCATCGGTGGCTGCGTGCTTGGGGTGATCTTCCTCGGGCTTGGCCTTT
TCATCCGTCACAGGAGTCAGAAAGGACCTCGAGGCCCTCCTCCAGCAGG
GCTCCTGCAGTGA
GFP (SEQ ID NO: 13)

ATGGTGAGCAAGGGCGAGGAGCTGTTCACCGGGGTGGTGCCCATCCTG
GTCGAGCTGGACGGCGACGTAAACGGCCACAAGTTCAGCGTGTCCGGC
GAGGGCGAGGGCGATGCCACCTACGGCAAGCTGACCCTGAAGTTCATCT
GCACCACCGGCAAGCTGCCCGTGCCCTGGCCCACCCTCGTGACCACCCT
GACCTACGGCGTGCAGTGCTTCAGCCGCTACCCCGACCACATGAAGCAG
CACGACTTCTTCAAGTCCGCCATGCCCGAAGGCTACGTCCAGGAGCGCA
CCATCTTCTTCAAGGACGACGGCAACTACAAGACCCGCGCCGAGGTGAA
GTTCGAGGGCGACACCCTGGTGAACCGCATCGAGCTGAAGGGCATCGA
CTTCAAGGAGGACGGCAACATCCTGGGGCACAAGCTGGAGTACAACTAC
AACAGCCACAACGTCTATATCATGGCCGACAAGCAGAAGAACGGCATCA
AGGTGAACTTCAAGATCCGCCACAACATCGAGGACGGCAGCGTGCAGCT
CGCCGACCACTACCAGCAGAACACCCCCATCGGCGACGGCCCCGTGCTG
CTGCCCGACAACCACTACCTGAGCACCCAGTCCGCCCTGAGCAAAGACC
CCAACGAGAAGCGCGATCACATGGTCCTGCTGGAGTTCGTGACCGCCGC
CGGGATCACTCTCGGCATGGACGAGCTGTACAAGTAA

293T cells (obtained from RIKEN BioResource Center) were used as host cells to which the above-described expression vectors were to be introduced. First, the 293 T cells were seeded in a 24-well plate at a density of 2 × 10⁵ cells/500 µl/well. Next, the expression vectors were introduced to the 293T cells using a PEI max solution obtained by dissolving a transfection reagent (PEI max, Cosmo Bio Co., Ltd.) in purified water at a concentration of 2 mg/ml. Specifically, a mixture of 2 µl of the PEI max solution and 50 µl of a serum-free medium (OPTI-MEM®, GIBCO) was added to 50 µl of a serum-free medium containing 0.26 µg of the I-A^{k} α expression vector, 0.26 µg of the I-A^{k} β expression vector, and 0.26 µg of the GFP expression vector. Then, they were mixed together, whereby a transfection reagent was prepared. Thereafter, the transfection reagent was added to each well to introduce the expression vectors to the 293 T cells. After the introduction, the 293 T cells were cultured in a DMEM medium at 37°C for 12 hours. Next, each of HEL peptides shown in Table 3 below was added to the wells at a concentration of 20 µmol/l. The 293 T cells were cultured for another 24 hours under the above-described culture conditions, whereby the 293 T cells were pulsed with the HEL peptide. The cultured 293 T cells were collected from the wells. Thereafter, the collected 293 T cells were used in antibody detection to be described below. The HEL peptides used in the present example and the following examples were obtained from SCRUM Inc., Eurofins Genomics K.K., or GenScript. In the HEL protein, a T-cell epitope presented by the MHC class II molecule I-A^{k} is a peptide consisting of the amino acid sequence of SEQ ID NO: 1 (DGSTDYGILQINSR). Thus, among the HEL peptides shown in Table 3 below, HEL peptide 41-70 corresponds to the immunizing peptide.

**[Table 3]**

| | Amino acid sequence |
|---|---|
| HEL peptide 1-30 (SEQ ID NO: 14) | KVFGRCELAAAMKRHGLDNYRGYSLGNWVC |
| HEL peptide 41-70 (SEQ ID NO: 15) | QATNRNTDGSTDYGILQINSRWWCNDGRTP |
| HEL peptide 48-61 (SEQ ID NO: 16) | DGSTDYGILQINSR |

### (2) Acquisition of antibody

100 µg of a HEL protein (Sigma) was mixed with a complete Freund's adjuvant (CFA, Sigma), and the resultant mixture was administered subcutaneously to mice (B10.A strain). Two weeks after the administration, whole blood was collected from each mouse, and serum was collected from the whole blood.

### (3) Antibody detection

The serum collected from each of the HEL protein-immunized mice was diluted 100-fold with a HANKS' BALANCED SALT SOLUTION (HBSS, Sigma) to obtain a diluted serum. Next, 10 µl of the diluted serum was added to the 293 T cells obtained in the above item (1) to cause a reaction. The 293 T cells after the reaction were washed, and then stained with an APC-labeled anti-mouse IgG (H+L) antibody (code-number: 715-136-150, Jackson ImmunoResearch). Thereafter, the stained 293 T cells were analyzed by flow cytometry (FACSCalibur™, Becton Dickinson) (peptide non-added groups). As peptide added groups, the HEL peptides shown in Table 3 above were added to the diluted serums, respectively, at a concentration of 40 µmol/l. Then, the diluted serums containing the respective HEL peptides were added to the 293 T cells pulsed with the corresponding HEL peptides to cause a reaction. Except for the above, the peptide added groups were analyzed by the flow cytometry analysis in the same manner.

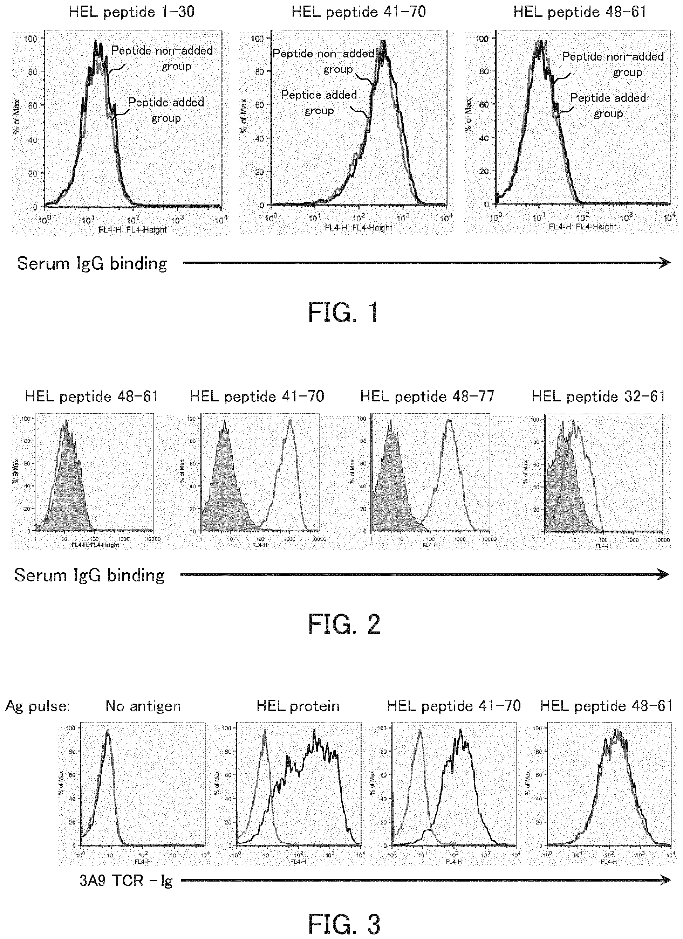

The results obtained are shown in FIG. 1. FIG. 1 shows histograms, each showing the amount of binding of an IgG antibody. In FIG. 1, the horizontal axis indicates the amount of binding of an IgG antibody in the serum, and the vertical axis indicates the cell counts. In FIG. 1, the type of the HEL peptide is shown above each histogram. As can be seen from FIG. 1, in the peptide non-added groups, the serum IgG binding was observed with respect to the 293 T cells pulsed with the HEL peptide 41-70 longer than the T-cell epitope presented by I-A^{k}, whereas the serum IgG binding was not observed with respect to the 293 T cells pulsed with the HEL peptide 48-61 including only the T-cell epitope presented by I-A^{k} and the 293 T cells pulsed with the HEL peptide 1-30 not including the T-cell epitope. Also, in the peptide added groups, similar results were obtained, namely, the serum IgG binding was observed with respect to the 293 T cells pulsed with the HEL peptide 41-70 longer than the T-cell epitope presented by I-A^{k}, whereas the serum IgG binding was not observed with respect to the 293 T cells pulsed with the HEL peptide 48-61 including only the T-cell epitope presented by I-A^{k} and the 293 T cells pulsed with the HEL peptide 1-30 not including the T-cell epitope. These results demonstrate that immunization with an HEL protein can induce production of an antibody to a complex of a peptide longer than a T-cell epitope (i.e., an immunizing peptide) and an MHC class II molecule.

### (4) Antibody binding sites

Peptide non-added groups were analyzed by flow cytometry in the same manner as in the above item (3), except that, instead of the HEL peptides shown in Table 3 above, the HEL peptide 41-70, the HEL peptide 48-61, and HEL peptides shown in Table 4 below were used. As controls, flow cytometry analysis was performed in the same manner, except that the I-A^{k} α expression vector and the I-A^{k} β expression vector were not introduced to the 293 T cells. It is to be noted that the HEL peptide 32-61, the HEL peptide 41-70, and the HEL peptide 48-77 correspond to the immunizing peptides. The HEL peptide 32-61 is a peptide including the T-cell epitope and the upstream antibody-inducing portion. The HEL peptide 41-70 is a peptide including the T-cell epitope, the upstream antibody-inducing portion, and the downstream antibody-inducing portion. The HEL peptide 48-77 is a peptide including the T-cell epitope and the downstream antibody-inducing portion.

**[Table 4]**

| | Amino acid sequence |
|---|---|
| HEL peptide 32-61 (SEQ ID NO: 17) | AKFESNFNTQATNRNTDGSTDYGILQINSR |
| HEL peptide 48-77 (SEQ ID NO: 18) | DGSTDYGILQINSRWWCNDGRTPGSRNLCN |

The results obtained are shown in FIG. 2. FIG. 2 shows histograms, each showing the amount of binding of an IgG antibody. In FIG. 2, the horizontal axis indicates the amount of binding of an IgG antibody in the serum, and the vertical axis indicates the cell counts. In FIG. 2, the type of the HEL peptide is shown above each histogram. The histograms plotted with the solid line show the results obtained regarding the peptide non-added groups, whereas the shaded histograms show the results obtained regarding the controls. As can be seen from FIG. 2, the serum IgG binding was not observed with respect to the 293 T cells not expressing the MHC class II molecule. Also, as can be seen from FIG. 2, the serum IgG binding was observed with respect to the 293 T cells pulsed with the HEL peptide 32-61, the HEL peptide 41-70, and the HEL peptide 48-77, which are all longer than the T-cell epitope presented by I-A^{k}, whereas the serum IgG binding was not observed with respect to the 293 T cells pulsed with the HEL peptide 48-61 including only the T-cell epitope presented by I-A^{k}. Moreover, the mean fluorescent intensity obtained when the HEL peptide 48-77 including the downstream antibody-inducing portion was used was higher than the mean fluorescent intensity obtained when the HEL peptide 32-61 including the upstream antibody-inducing portion was used. These results demonstrate that immunization with a HEL protein can induce antibody production to all the following complexes: a complex of a peptide including a T-cell epitope and an upstream antibody-inducing portion with an MHC class II molecule; a complex of a peptide including the T-cell epitope and a downstream antibody-inducing portion with the MHC class II molecule; and a complex of a peptide including the T-cell epitope, the upstream antibody-inducing portion, and the downstream antibody-inducing portion with the MHC class II molecule. These results also suggest that, in the case of a HEL protein, an immunizing peptide including a T-cell epitope and a downstream antibody-inducing portion can induce a higher degree of antibody production than an immunizing peptide including the T-cell epitope and an upstream antibody-inducing portion.

### (Example 1B)

The present example examined whether the antibody produced in Example 1A inhibits the binding of a TCR that recognizes a T-cell epitope-MHC class II molecule complex to an immunizing peptide-MHC class II molecule complex.

### (1) Preparation of soluble single-chain TCR

From 3A9 T cell hybridomas (obtained from Laboratory of Vaccine Materials, National Institutes of Biomedical Innovation) expressing a TCR that recognizes a complex of a peptide consisting of the amino acid sequence of SEQ ID NO: 1 (the T-cell epitope) and I-A^{k} (the MHC class II molecule), the TCR was cloned. Specifically, from cDNA derived from the 3A9 T cell hybridomas, a polynucleotide encoding the following α-chain and β-chain of 3A9 TCR linked by a GGGGS linker was cloned into a pME18S vector. Thus, a 3A9 TCR expression vector was produced. The vector was designed so that the Fc domain of human IgG is added to the C terminus of an expressed protein. Thus, by introducing the expression vector to a host, a soluble single-chain TCR composed of 3A9 TCR and Fc bound thereto is expressed (3A9 scTCR-Fc).
α and β-chains of single-chain 3A9 TCR (SEQ ID NO: 19)

ATGCTCCTGGCACTCCTCCCAGTGCTGGGGATACACTTTGTCCTGAGAG
ATGCCCAAGCTCAGTCAGTGACACAGCCCGATGCTCGCGTCACTGTCTC
TGAAGGAGCCTCTCTGCAGCTGAGATGCAAGTATTCCTACTCTGGGACG
CCTTATCTGTTCTGGTATGTCCAGTACCCGCGGCAGGGGCTGCAGCTGC
TCCTCAAGTACTATTCCGGAGACCCAGTGGTTCAAGGAGTGAATGGCTT
TGAGGCTGAGTTCAGCAAGAGTAACTCTTCCTTCCACCTGCGGAAAGCC
TCCGTGCACTGGAGCGACTCGGCTGTGTACTTCTGTGCTTTAAATTCTGG
AGGAAGCAATGCAAAGCTAGCCTTCGGGAAAGGCTCTAAACTCTCTGTT
AAATCAAACGGCGGAGGCGGATCAGGAGGAGGAGGATCAGGCGGAGGA
GGATCAGCAGTCACCCAAAGCCCAAGAAACAAGGTGGCAGTAACAGGA
GGAAAGGTGACATTGAGCTGTAATCAGACTAATAACCACAACAACATGTA
CTGGTATCGGCAGGACACGGGGCATGGGCTGAGGCTGATCCATTATTCA
TATGGTGCTGGCAGCACTGAGAAAGGAGATATCCCTGATGGATACAAGG
CCTCCAGACCAAGCCAAGAGAACTTCTCCCTCATTCTGGAGTTGGCTAC
CCCCTCTCAGACATCAGTGTACTTCTGTGCCAGCGGTGATGAATCGGCC
CTACCGGAGCTCTACTTTGGTGAAGGCTCAAAGCTGACAGTGCTGGAGG
ATCTGAGAAATGTGACTCCACCCAAGGTCTCCTTGTTTGAGCCATCAAAA
GCAGAGATTGCAAACAAACAAAAGGCTACCCTCGTGTGCTTGGCCAGGG
GCTTCTTCCCTGACCACGTGGAGCTGAGCTGGTGGGTGAATGGCAAGGA
GGTCCACAGTGGGGTCAGCACGGACCCTCAGGCCTACAAGGAGAGCAA
TTATAGCTACTGCCTGAGCAGCCGCCTGAGGGTCTCTGCTACCTTCTGGC
ACAATCCTCGCAACCACTTCCGCTGCCAAGTGCAGTTCCATGGGCTTTC
AGAGGAGGACAAGTGGCCAGAGGGCTCACCCAAACCTGTCACACAGAA
CATCAGTGCAGAGGCCTGGGGCCGAGCAGAC

Next, the 293 T cells were cultured for 96 hours under the same culture conditions as in the item (1) in Example 1A, except that the 3A9 TCR expression vector was introduced instead of the I-A^{k} α expression vector and the I-A^{k} β expression vector. After the culture, the supernatant was collected, and the collected supernatant was used as the soluble single-chain TCR.

### (2) Preparation of HEL peptide-presenting cells

LK35.2 cells expressing I-A^{k} (the MHC class II molecule) were seeded in a 24-well plate at a density of 1 × 10⁵ cells/1 ml/well. Next, the HEL protein was added to the wells at a concentration of 200 µg/ml, or the HEL peptide 41-70 or the HEL peptide 48-61 was added to the wells at a concentration of 20 µmol/l. The LK35.2 cells were pulsed with the HEL protein or the HEL peptide by culturing the HEL protein-added group for another 48 hours and the HEL peptide added groups for another 24 hours under the above-described culture conditions. The cultured LK35.2 cells were collected from the wells. Thereafter, the collected LK35.2 cells were used in inhibition of TCR recognition by an antibody to be described below.

### (3) Inhibition of TCR recognition by antibody

Serum collected from each of the HEL protein-immunized mice was diluted 10-fold with HBSS to obtain a diluted serum. Next, 10 µl of the diluted serum was added to the LK35.2 cells obtained in the above item (2) to cause a reaction. Further, a dimer of 20 µl of the supernatant obtained in the above item (1) and 10 µl of an APC-labeled anti-human IgG Fc antibody (code number:109-136-098, Jackson ImmunoResearch) diluted 100-fold was formed beforehand, and the LK35.2 cells after the reaction were reacted with the dimer. Then, the LK35.2 cells were analyzed by flow cytometry. As Control 1, the flow cytometry analysis was performed in the same manner, except that serums collected from non-immunized mice were used instead of the serums of the HEL protein-immunized mice. As Control 2, the flow cytometry analysis was performed in the same manner, except that the HEL protein or the HEL peptide was not added.

The results obtained are shown in FIG. 3. FIG. 3 shows histograms, each showing the amount of binding of the soluble single-chain TCR. In FIG. 3, the horizontal axis indicates the amount of binding of the soluble single-chain TCR, and the vertical axis indicates the cell counts. In FIG. 3, the absence of the HEL protein or the HEL peptides (No antigen), the HEL protein, and the type of the HEL peptide are shown above the respective histograms. The histograms plotted with the black solid line show the results obtained regarding Control 1, and the histograms plotted with the gray solid line show the results obtained when the serums of the respective types of the immunized mice were added. As can be seen from FIG. 3, in Control 2, the binding of the soluble single-chain TCR was not observed. When the LK35.2 cells pulsed with the HEL protein or each of the HEL peptides were reacted with the serums of the non-immunized mice, binding of the soluble single-chain TCR was observed. Further, when the LK35.2 cells pulsed with the HEL peptide 48-61 were reacted with the serums of the HEL-immunized mice, binding of the soluble single-chain TCR was observed. In contrast, when the LK35.2 cells pulsed with the HEL protein or the HEL peptide 41-70 were reacted with the serums of the HEL-immunized mice, binding of the soluble single-chain TCR was not observed. These results demonstrate that immunization with a HEL protein can induce production of an antibody that inhibits the binding of a TCR that recognizes a T-cell epitope-MHC class II molecule complex to an immunizing peptide-MHC class II molecule complex.

### (4) Purification of antibody in serum

An IgG antibody was purified from the serums of the HEL protein-immunized mice. Specifically, the IgG antibody was purified by diluting 100 µl of the serum 10-fold with phosphate buffered saline (PBS) and applying the thus-obtained sample to affinity chromatography using a Profinia (Bio Rad). In the affinity chromatography, a column containing protein A (Bil-Scale mini UNO Sphere SUPrA Cartridege, Bio Rad) was used. Then, the sample was supplied to the column, thereby causing a reaction between the IgG antibody in the serum and the protein A in the column. After the reaction, the column was washed once with PBS. Then, 0.1 mol/l Glycine-HCl (pH 2.8) was added to the column, and the IgG antibody was collected from the column.

### (5) Inhibition of TCR recognition by purified IgG antibody

Spleen cells were prepared from each of the mice by an ordinary method. Then, the spleen cells, 3A9 T cell hybridomas, HEL peptide 41-70, and the IgG antibody were seeded in a 96 -well plate at a density/concentration of 1 × 10⁵ spleen cells, 1 × 10⁵ 3A9 T cell hybridomas, 10 µmol/l HEL peptide 41-70, and a predetermined concentration (2.6, 7.8, or 23.4 µg/ml) of IgG antibody/200 µl/well. After the seeding, the cells in the wells were cultured at 37°C for 24 hours using an RPMI medium. After the culture, the supernatant was collected. Then, the concentration of IL-2 in the supernatant was measured by an ELISA method. As a control, the concentration of IL-2 in the supernatant was measured in the same manner, except that the IgG antibody was not added. In the ELISA method, an affinity purified anti-mouse IL-2 (clone name: JES6.1A12, eBioscience) was used as a capture antibody for capturing IL-2, a biotin-anti-mouse IL-2 (clone name: JES6.5H4, eBioscience) was used as a detection antibody for detecting IL-2, and the binding of the detection antibody was detected using an avidin-HRP (SIGMA).

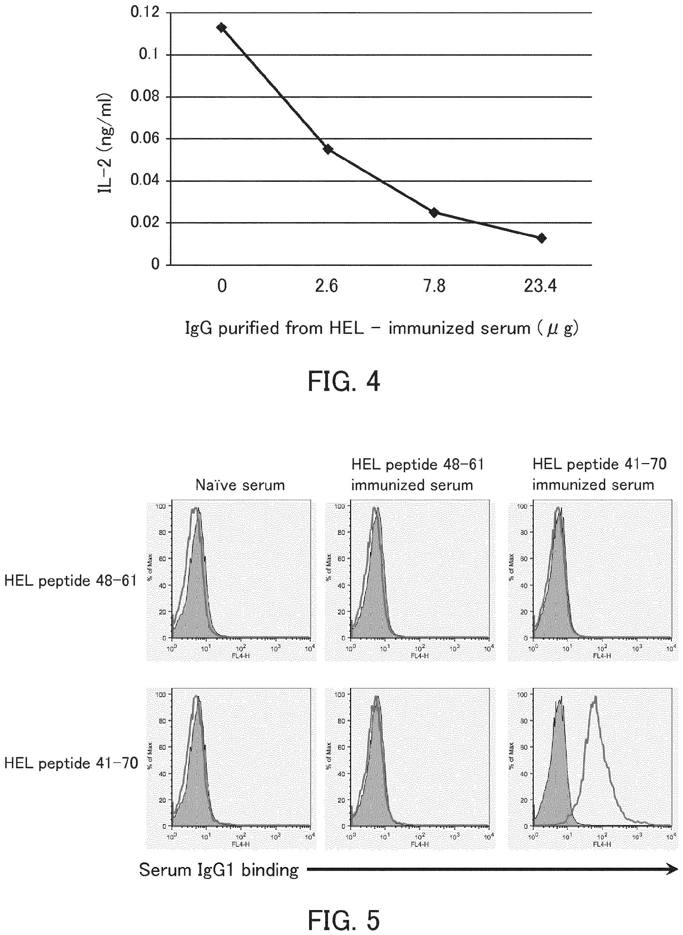

The results obtained are shown in FIG. 4. FIG. 4 is a graph showing the amount of the IL-2 produced. In FIG. 4, the horizontal axis indicates the concentration of the IgG antibody, and the vertical axis indicates the concentration of the IL-2. As can be seen from FIG. 4, the concentration of the IL-2 decreased in an IgG antibody concentration dependent manner. The concentration of the IL-2 correlates with the degree of the activation of the 3A9 T cell hybridomas, i.e., the degree to which the 3A9 TCR of the 3A9 T cell hybridomas can bind to the T-cell epitope-MHC class II molecule complex. Thus, these results demonstrate that immunization with a HEL protein can induce production of an IgG antibody that inhibits the binding of a TCR that recognizes a T-cell epitope-MHC class II molecule complex.

### [Example 2]

The present example examined whether immunization with an immunizing peptide induces antibody production to an immunizing peptide-MHC class II molecule complex and whether the antibody inhibits the binding of a TCR that recognizes a T-cell epitope-MHC class II molecule complex.

### (Example 2A)

The present example examined whether immunization with an immunizing peptide induces antibody production to an immunizing peptide-MHC class II molecule complex.

### (1) Acquisition of antibody

30 nmol of the HEL peptide 41-70 or the HEL peptide 48-61 was mixed with a complete Freund's adjuvant. Each of the thus-obtained mixture was administered subcutaneously to mice (B10.A strain). Two weeks after the administration, whole blood was collected from each mouse, and serum was collected from the whole blood.

### (2) Preparation of HEL peptide-presenting cells

LK35.2 cells were pulsed with the HEL peptide in the same manner as in the item (2) in Example 1B, except that the HEL peptide 41-70 or the HEL peptide 48-61 was added instead of the HEL protein, the HEL peptide 41-70, and the HEL peptide 48-61. The cultured LK35.2 cells were collected from the wells. Thereafter, the collected LK35.2 cells were used in antibody detection to be described below.

### (3) Antibody detection

Flow cytometry analysis was performed in the same manner as in the item (3) in Example 1A, except that the LK35.2 cells in the item (2) in Example 2A were used instead of the 293 T cells and that an Alexa® 647-labeled anti-mouse IgG1 antibody (catalog number: A21240, Invitrogen) was used instead of the APC-labeled anti-mouse IgG (H+L) antibody (peptide added groups). As Control 1, the flow cytometry analysis was performed in the same manner, except that serums collected from non-immunized mice were used instead of the serums of the HEL peptide-immunized mice. As Control 2, the flow cytometry analysis was performed in the same manner, except that the HEL peptide was not added (peptide non-added groups).

The results obtained are shown in FIG. 5. FIG. 5 shows histograms, each showing the amount of binding of an IgG1 antibody. In FIG. 5, the horizontal axis indicates the amount of binding of an IgG1 antibody in the serum, and the vertical axis indicates the cell counts. In FIG. 5, the type of the serum is shown above each histogram, and the type of the HEL peptide used to pulse the LK35.2 cells is shown on the left of the rows of the histograms. In FIG. 5, the histograms plotted with the solid line show the results obtained regarding the peptide added groups, and the shaded histograms show the results obtained regarding Control 2 (the peptide non-added groups). As can be seen from FIG. 5, in Control 1 (Naive serum) and the serums of the HEL peptide 48-61-immunized mice, the IgG1 binding was not observed with respect to the LK35.2 cells pulsed with the HEL peptide 41-70 or the HEL peptide 48-61. In contrast, in the serums of the HEL peptide 41-70-immunized mice, the IgG1 binding was not observed with respect to the LK35.2 cells pulsed with the HEL peptide 48-61, whereas the IgG1 binding was observed with respect to the LK35.2 cells pulsed with the HEL peptide 41-70. These results demonstrate that immunization with the HEL peptide 41-70, i.e., an immunizing peptide, can induce antibody production to a complex of the immunizing peptide and an MHC class II molecule.

### (Example 2B)

The present example examined whether the antibody produced in Example 2A inhibits the binding of a TCR that recognizes a T-cell epitope-MHC class II molecule complex to the immunizing peptide-MHC class II molecule complex.

### (1) Preparation of HEL peptide-presenting cells

LK35.2 cells were pulsed with the HEL protein in the same manner as in the item (2) in Example 1B, except that the HEL protein was added instead of the HEL protein, the HEL peptide 41-70, and the HEL peptide 48-61. The cultured LK35.2 cells were collected from the wells. Thereafter, the collected LK35.2 cells were used in inhibition of TCR recognition by an antibody to be described below.

### (2) Inhibition of TCR recognition by antibody

Flow cytometry analysis was performed in the same manner as in the item (3) in Example 1B, except that serums of the HEL peptide 41-70-immunized mice or serums of the HEL peptide 48-61-immunized mice were used in addition to the serums of the HEL protein-immunized mice and that the LK35.2 cells obtained in the item (1) in Example 2B were used instead of the LK35.2 cells obtained in the item (2) in Example 1B. As Control 1, the flow cytometry analysis was performed in the same manner, except that serums collected from non-immunized mice were used instead of the serums of the HEL protein-immunized mice, the serums of the HEL peptide 41-70-immunized mice, and the serums of the HEL peptide 48-61-immunized mice. As Control 2, the flow cytometry analysis was performed in the same manner, except that the HEL protein was not added.

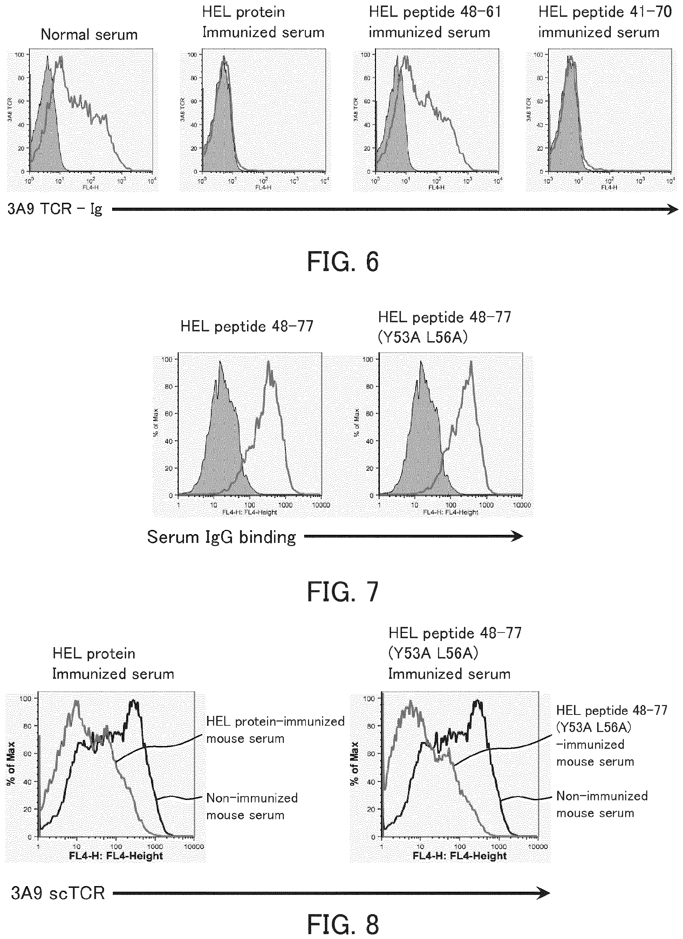

The results obtained are shown in FIG. 6. FIG. 6 shows histograms, each showing the amount of binding of a soluble single-chain TCR. In FIG. 6, the horizontal axis indicates the amount of binding of the soluble single-chain TCR, and the vertical axis indicates the cell counts. In FIG. 6, the type of the serum is shown above each histogram. The histograms plotted with the solid line show the results obtained when the LK35.2 cells pulsed with the HEL protein were used, and the shaded histograms show the results obtained regarding Control 2. As can be seen from FIG. 6, in Control 2, binding of the soluble single-chain TCR was not observed in any type of the serum. When the LK35.2 cells were reacted with the serums of Control 1 (Normal Serum) and the serums of the HEL peptide 48-61-immunized mice, binding of the soluble single-chain TCR was observed. In contrast, when the LK35.2 cells were reacted with the serums of the HEL protein-immunized mice or the serums of the HEL peptide 41-70-immunized mice, binding of the soluble single-chain TCR was not observed. These results demonstrate that immunization with a HEL protein and the HEL peptide 41-70, i.e., an immunizing peptide, can induce production of an antibody that inhibits the binding of a TCR that recognizes a T-cell epitope-MHC class II molecule complex to an immunizing peptide-MHC class II molecule complex.

### [Example 3]

The present example examined whether the IgG antibody in the serums of the HEL protein-immunized mice binds to a complex of an immunizing peptide including a substituted T-cell epitope in which amino acid residues recognized by a TCR in the amino acid sequence of an unsubstituted T-cell epitope are substituted and an MHC class II molecule.

### (1) Preparation of HEL peptide-presenting cells

293 T cells were pulsed with HEL peptides in the same manner as in the item (1) in Example 1A, except that the above HEL peptide 48-77 and the following HEL peptide 48-77 (Y53A L56A) were used instead of the HEL peptides shown in Table 3 above. The cultured 293 T cells were collected from the wells. Thereafter, the collected 293 T cells were used in antibody detection to be described below. The HEL peptide 48-77 (Y53A L56A) is an immunizing peptide including a substituted T-cell epitope obtained by, in the peptide consisting of the amino acid sequence of SEQ ID NO: 1 (the T-cell epitope), substituting the 6th tyrosine and the 9th leucine, which are amino acid residues known to be recognized by the TCR, with alanine.

### (2) Antibody detection

Flow cytometry analysis was performed in the same manner as in the item (3) in Example 1A, except that the 293 T cells obtained in the item (1) in Example 3 were used instead of the 293 T cells obtained in the item (1) in Example 1A (the peptide added groups). As a control, the flow cytometry analysis was performed in the same manner, except that LK35.2 cells not pulsed with the HEL peptides were used instead of the 293 T cells (the peptide non-added group).

FIG. 7 shows histograms, each showing the amount of binding of an IgG antibody. In FIG. 7, the horizontal axis indicates the amount of binding of an IgG antibody in the serum, and the vertical axis indicates the cell counts. In FIG. 7, the type of the HEL peptide used to pulse the 293 T cells is shown above each histogram. The shaded histograms show the result obtained regarding the control (the peptide non-added group), and the histograms plotted with the solid line show the results obtained regarding the peptide added groups. As can be seen from FIG. 7, in the control, the serum IgG binding was not observed. In contrast, the serum IgG binding was observed with respect to the 293 T cells pulsed with the HEL peptide 48-77 and the 293 T cells pulsed with the HEL peptide 48-77 (Y53A L56A). These results demonstrate that immunization with a HEL protein can induce antibody production to a complex of an immunizing peptide including a substituted T-cell epitope in which amino acid residues recognized by the TCR are substituted.

### [Example 4]

The present example examined whether an antibody produced as a result of immunization with an immunizing peptide including a substituted T-cell epitope obtained by substituting amino acid residues recognized by a TCR in the amino acid sequence of an unsubstituted T-cell epitope inhibits the binding of the TCR that recognizes a T-cell epitope-MHC class II molecule complex.

### (1) Acquisition of antibody

30 nmol of the HEL peptide 48-77 (Y53A L56A) was mixed with a complete Freund's adjuvant, and the resultant mixture was administered subcutaneously to mice (B10.A strain). Two weeks after the administration, whole blood was collected from each mouse, and serum was collected from the whole blood.

### (2) Preparation of HEL peptide-presenting cells

LK35.2 cells were pulsed with the HEL protein in the same manner as in the item (2) in Example 1B, except that the HEL protein was added instead of the HEL protein, the HEL peptide 41-70, and the HEL peptide 48-61. The cultured LK35.2 cells were collected from the wells. Thereafter, the collected LK35.2 cells were used in inhibition of TCR recognition by an antibody to be described below.

### (3) Inhibition of TCR recognition by antibody

Flow cytometry analysis was performed in the same manner as in the item (3) in Example 1B, except that serums of the HEL peptide 48-77 (Y53A L56A)-immunized mice were used in addition to the serums of the HEL protein-immunized mice and that the LK35.2 cells obtained in the item (2) in Example 4 were used instead of the LK35.2 cells obtained in the item (2) in Example 1B. As controls, the flow cytometry analysis was performed in the same manner, except that serums collected from non-immunized mice were used instead of the serums of the HEL protein-immunized mice and the serums of the HEL peptide 48-77 (Y53A L56A)-immunized mice.

The results obtained are shown in FIG. 8. FIG. 8 shows histograms, each showing the amount of binding of a soluble single-chain TCR. In FIG. 8, the horizontal axis indicates the amount of binding of the soluble single-chain TCR, and the vertical axis indicates the cell counts. In FIG. 8, the type of the serum is shown above each histogram. As can be seen from FIG. 8, when the LK35.2 cells were reacted with the serums of the control (the serums of the non-immunized mice), binding of the soluble single-chain TCR was observed. In contrast, when the LK35.2 cells were reacted with the serums of the HEL protein-immunized mice and the serums of the HEL peptide 48-77 (Y53A L56A)-immunized mice, binding of the soluble single-chain TCR was not observed. These results demonstrate that immunization with an immunizing peptide including a substituted T-cell epitope obtained by substituting amino acid residues recognized by a TCR in the amino acid sequence of an unsubstituted T-cell epitope can induce production of an antibody that inhibits the binding of the TCR that recognizes a T-cell epitope-MHC class II molecule complex.

### [Example 5]

The present example examined whether immunization with an immunizing peptide induces antibody production to an immunizing peptide-MHC class II molecule complex.

### (1) Preparation of PLP peptide-presenting cells

From mouse spleen cDNA, polynucleotides encoding the α-chain and the β-chain of a mouse MHC class II molecule (I-A^{s}) shown below were cloned into pME18S vectors, respectively. Thus, a mouse MHC class II molecule (I-A^{s}) α-chain expression vector (I-A^{s} α expression vector) and a mouse MHC class II molecule (I-A^{s}) β-chain expression vector (I-A^{s} β expression vector) were produced.
α-chain of mouse MHC class II molecule (I-A^{s}) (SEQ ID NO: 22) 
ATGCCGCGCAGCAGAGCTCTGATTCTGGGGGTCCTCGCCCTGACCACCA
TGCTCAGCCTCTGTGGAGGTGAAGACGACATTGAGGCCGACCACGTAGG
CGTCTATGGTACAACTGTATATCAGTCTCCTGGAGACATTGGCCAGTACA
CACATGAATTTGATGGTGATGAGTGGTTCTATGTGGACTTGGATAAGAAG
GAGACTATCTGGATGCTTCCTGAGTTTGGCCAATTGACAAGCTTTGACCC
CCAAGGTGGACTGCAAAACATAGCTACAGGAAAATACACCTTGGGAATC
TTGACTAAGAGGTCAAATTCCACCCCAGCTACCAATGAGGCTCCTCAAG
CGACTGTGTTCCCCAAGTCCCCTGTGCTGCTGGGTCAGCCCAACACCCT
TATCTGCTTTGTGGACAACATCTTCCCTCCTGTGATCAACATCACATGGC
TCAGAAATAGCAAGTCAGTCACAGACGGCGTTTATGAGACCAGCTTCCT
TGTCAACCGTGACCATTCCTTCCACAAGCTGTCTTATCTCACCTTCATCC
 
CTTCTGACGATGATATTTATGACTGCAAGGTGGAGCACTGGGGCCTGGA
GGAGCCGGTTCTGAAACACTGGGAACCTGAGATTCCAGCCCCCATGTCA
GAGCTGACAGAGACTGTGGTGTGTGCCCTGGGGTTGTCTGTGGGCCTTG
TGGGCATCGTGGTGGGCACCATCTTCATCATTCAAGGCCTGCGATCAGG
TGGCACCTCCAGACACCCAGGGCCTTTATGA
β-chain of mouse MHC class II molecule (I-A^{s}) (SEQ ID NO: 23)

ATGGCTCTGCAGATCCCCAGCCTCCTCTCGGCTGCTGTGGTGGTGC
TGATGGTGCTGAGCAGCCCAGGGACTGAGGGCGGAGACTCCGAAAGGC
ATTTCGTGTTCCAGTTCAAGGGCGAGTGCTACTTCACCAACGGGACGCA
GCGCATACGATCTGTGGACAGATACATCTACAACCGGGAGGAGTACCTG
CGCTTCGACAGCGACGTGGGCGAGTACCGCGCGGTGACCGAGCTGGGG
CGGCCAGACCCCGAGTACTACAATAAGCAGTACCTGGAGCAAACGCGGG
CCGAGCTGGACACGGTGTGCAGACACAACTACGAGGGGGTGGAGACCC
ACACCTCCCTGCGGCGGCTTGAACAGCCCAATGTCGTCATCTCCCTGTC
CAGGACAGAGGCCCTCAACCACCACAACACTCTGGTCTGCTCAGTGACA
GATTTCTACCCAGCCAAGATCAAAGTGCGCTGGTTCCGGAATGGCCAGG
AGGAGACGGTGGGGGTCTCATCCACACAGCTTATTAGGAATGGGGACTG
GACCTTCCAGGTCCTGGTCATGCTGGAGATGACCCCTCGGCGGGGAGAG
GTCTACACCTGCCACGTGGAGCATCCGAGCCTGAAGAGCCCCATCACTG
TGGAGTGGAGGGCACAGTCTGAGTCTGCCCGGAGCAAGATGTTGAGCG
GCATCGGGGGCTGCGTGCTTGGGGTGATCTTTCTCGGGCTTGGCCTTTT
CATCCGTCACAGGAGTCAGAAAGGACCTCGAGGCCCTCCTCCTGCAGGG
CTCCTGCAGTGA

Next, the expression vectors were introduced to the 293 T cells in the same manner as in the item (1) in Example 1A, except that the I-A^{s} α expression vector and the I-A^{s} β expression vector were used instead of the I-A^{k} α expression vector and the I-A^{k} β expression vector. Further, except that myelin proteolipid protein (PLP) peptide 136-151 or PLP peptide 139-151 shown in Table 5 below was added instead of the HEL peptide, the 293 T cells were pulsed with the PLP peptide in the same manner as in the item (1) in Example 1A. In the PLP peptides used below, the PLP peptide 139-151 (SEQ ID NO: 20) shown in Table 5 was used as a T-cell epitope. The PLP peptide 139-151 is a peptide consisting of an amino acid sequence obtained by substituting cysteine as the 2nd amino acid in the amino acid sequence of the above SEQ ID NO: 7 with serine. In the PLP peptide 136-151 (SEQ ID NO: 21), the 3rd amino acid from the N terminus was substituted from cysteine to serine in the upstream antibody-inducing portion, as indicated with the underline in Table 5. The cultured 293 T cells were collected from the wells. Thereafter, the collected 293 T cells were used in antibody detection to be described below. The PLP peptides used in the present example were obtained from Eurofins Genomics K.K. or GenScript. In the PLP protein, the T-cell epitope presented by the MHC class II molecule I-A^{s} is the PLP peptide 139-151 shown in Table 5. Thus, among the PLP peptides shown in Table 5, the PLP peptide 136-151 corresponds to the immunizing peptide.

**[Table 5]**

| | Amino acid sequence |
|---|---|
| PLP peptide 139-151 (SEQ ID NO: 20) | HSLGKWLGHPDKF |
| PLP peptide 136-151 (SEQ ID NO: 21) | RVSHSLGKWLGHPDKF |

### (2) Acquisition of antibody

Serums were collected in the same manner as in the item (2) in Example 1A, except that 30 nmol of the PLP peptide 139-151 or the PLP peptide 136-151 was used instead of the HEL protein and that the PLP peptide 139-151 or the PLP peptide 136-151 was administered to mice (B10.S strain or SJL) instead of the above-described mice (B10.A strain).

### (3) Antibody detection

Flow cytometry analysis was performed in the same manner as in the item (3) in Example 1A, except that serums of PLP peptide 139-151-immunized mice or serums of PLP peptide 136-151-immunized mice were used instead of the serums of the HEL protein-immunized mice and that the 293 T cells obtained in the item (1) in Example 5 were used instead of the 293 T cells obtained in the item (1) in Example 1A (the peptide added groups). As controls, the flow cytometry analysis was performed in the same manner, except that the 293 T cells were not pulsed with the respective PLP peptides (peptide non-added groups).

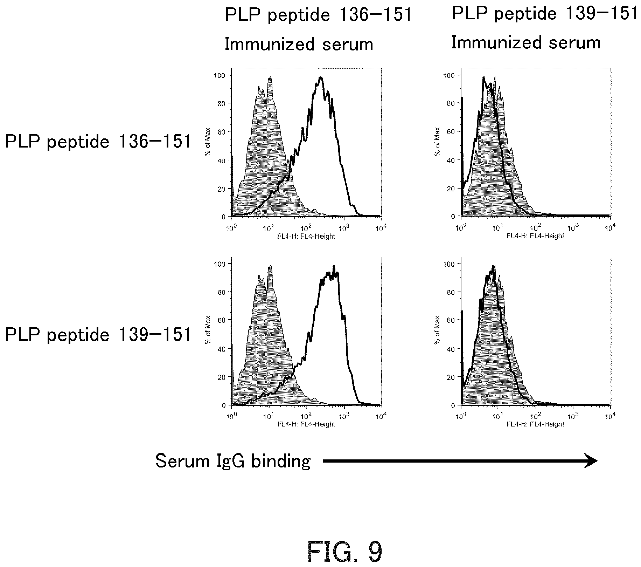

The results obtained are shown in FIG. 9. FIG. 9 shows histograms, each showing the amount of binding of an IgG antibody. In FIG. 9, the horizontal axis indicates the amount of binding of an IgG antibody in the serum, and the vertical axis indicates the cell counts. In FIG. 9, the type of the serum is shown above each histogram. In FIG. 9, the histograms plotted with the solid line show the results obtained regarding the peptide added groups, and the shaded histograms show the results obtained regarding the controls (the peptide non-added groups). As can be seen from FIG. 9, in the peptide non-added groups (controls), binding of the IgG antibody was not observed in any type of the serum. In the serums of the PLP peptide 139-151-immunized mice, the IgG binding was not observed with respect to the 293 T cells pulsed with the PLP peptide 136-151 or the PLP peptide 139-151. In contrast, in the serums of the PLP peptide 136-151-immunized mice, the IgG binding was observed with respect to the 293 T cells pulsed with the PLP peptide 136-151 and the 293 T cells pulsed with the PLP peptide 139-151. These results demonstrate that immunization with the PLP peptide 136-151, i.e., an immunizing peptide, can induce antibody production to a complex of the immunizing peptide and an MHC class II molecule.

### [Example 6]

The present example examined whether an antibody obtained by immunization with an immunizing peptide inhibits the binding of a TCR that recognizes a T-cell epitope-MHC class II molecule complex to an immunizing peptide-MHC class II molecule complex.

### (1) Preparation of PLP peptide-presenting cells

293 T cells pulsed with a PLP peptide were prepared in the same manner as in the item (1) in Example 5, except that the PLP peptide 136-151 was added at a concentration of 10 µmol/l.

### (2) Acquisition of IgG antibody

Serums were collected in the same manner as in the item (2) in Example 1A, except that: 30 nmol of the PLP peptide 136-151 was used instead of the HEL protein; mice (SJL/J strain) were used instead of the mice (B10.A strain); and whole bloods were collected from the mice on Day 14 to Day 30 after the administration. An IgG antibody was purified from the obtained serums using Protein A and Protein G (Bio-rad) in accordance with the protocols attached thereto.

### (3) Preparation of reporter T cells

Reporter T cells expressing a TCR (2E5 TCR) that recognizes a complex of the PLP peptide 139-151 (T-cell epitope) and I-A^{s} (MHC class II molecule) and adapted to express GFP upon TCR stimulation were prepared in the following manner.

First, the TCR α-chain gene and the TCR β-chain gene were deleted from the cells of the 43-1 cell line described in the following Reference Document using a CRISPR-Cas9 (clustered regularly interspaced short palindromic repeats/CRISPR associated proteins) system, and the cells were collected by flow cytometry. The collected cells were subjected to limiting dilution, and clones negative for the cell surface expression of the TCR were selected (first selected cells). Among the first selected cells, clones that do not express TCRs on cell surfaces when the TCR α-chain gene or the TCR β-chain gene is introduced thereto independently but express TCRs on cell surfaces when both the TCR α-chain and β-chain genes are introduced thereto were selected (second selected cells). The TCR α-chain and β-chain genes were introduced to the second selected cells. Then, the second selected cells were stimulated with an immobilized anti-CD3 antibody (clone name: 2C11, concentration for immobilization: 10 µg/ml) in the state where the second selected cells express TCRs on their surfaces, and clones expressing a reporter protein GFP well were selected. The thus-selected cells were used as TCR-deleted 43-1 cells.

Reference document: Makoto Ohtsuka et.al., "NFAM1, an immunoreceptor tyrosine-based activation motif-bearing molecule that regulates B cell development and signaling", PNAS, 2004, vol.101, No.21, pages 8126-8131

Next, a base sequence consisting of 170th to 1543rd bases (including the termination codon) in mRNA of the mouse CD4 molecule registered under NCBI Accession No. NM_013488.2 was cloned into a pMXs vector (provided by Dr. Toshio Kitamura in the Institute of Medical Science, the University of Tokyo). Thus, a CD4 expression vector was produced. The CD4 expression vector was transfected into a Plat-E cell to produce a retrovirus. The TCR deleted 43-1 cell was infected with the retrovirus. Thus, CD4 expression 43-1 cells stably expressing mouse CD4 were produced. Further, the 2E5 TCR α-chain gene (SEQ ID NO: 24, including the termination codon) and the 2E5 TCR β-chain gene (SEQ ID NO: 25, including the termination codon) were cloned into a pMXs vector. Thus, a 2E5 TCR expression vector was produced. Then, a retrovirus was produced in the same manner, except that the 2E5 TCR expression vector was used instead of the CD4 expression vector and that the CD4 expression 43-1 cells were used instead of the TCR deleted 43-1 cells. Using the thus-obtained retrovirus, 2E5 reporter T cells stably expressing 2E5 TCR were produced.
2E5 TCR α-chain gene (SEQ ID NO: 24) 
ATGAAGACATCCCTTCACACTGTATTCCTATTCTTGTGGCTGTGGATGGA
CTGGGAAAGCCATGGAGAGAAGGTCGAGCAACACCAGTCTACACTGAGT
GTTCGAGAGGGAGACAGCGCTGTCATCAACTGCACTTACACAGATACTG
 
CCTCATCATACTTCCCTTGGTACAAGCAAGAAGCTGGAAAGAGTCTCCAC
TTTGTGATAGACATTCGTTCAAATGTGGACAGAAAACAGAGCCAAAGAC
TTACAGTTTATTGGATAAGAAAGCCAAACGATTCTCCCTGCACATCACA
GCCACACAGCCTGAAGATTCAGCCATCTACTTCTGTGCAGCAAGCCCGG
GAGCTAACACTGGAAAGCTCACGTTTGGACACGGCACCATCCTTAGGGT
CCATCCAAACATCCAGAACCCAGAACCTGCTGTGTACCAGTTAAAAGATC
CTCGGTCTCAGGACAGCACCCTCTGCCTGTTCACCGACTTTGACTCCCA
AATCAATGTGCCGAAAACCATGGAATCTGGAACGTTCATCACTGACAAAA
CTGTGCTGGACATGAAAGCTATGGATTCCAAGAGCAATGGGGCCATTGC
CTGGAGCAACCAGACAAGCTTCACCTGCCAAGATATCTTCAAAGAGACC
AACGCCACCTACCCCAGTTCAGACGTTCCCTGTGATGCCACGTTGACCG
AGAAAAGCTTTGAAACAGATATGAACCTAAACTTTCAAAACCTGTCAGTT
ATGGGACTCCGAATCCTCCTGCTGAAAGTAGCGGGATTTAACCTGCTCAT
GACGCTGAGGCTGTGGTCCAGTTGA
2E5 TCR β-chain gene (SEQ ID NO: 25)

ATGGGTGCAAGACTGCTCTGCTGTGTAGCAGTTTGTCTGCTTGGGGCAG
GCTCTTTTGTTGCTGGAGTAACCCAGACTCCACGATACCTGGTCAAAGA
GGAAGGACAGAAAGCACACATGAGTTGTAGTCCTGAAAAGGGCACACT
GCCTTTTACTGGTATCAACAGAACCAGAAACAAGAACTTACATTTTGAT
TAACTTTCGGAATGAAGAAATTATGGAACAAACAGACTTGGTCAAGAAG
AGATTCTCAGCTAAGTGTTCCTCGAACTCACAGTGCATCCTGGAAATCCT
ATCCTCTGAAGAAGACGACTCAGCACTGTACCTCTGTGCCAGCAGCCTT
GTACGAGGGAACTCCGACTACACCTTCGGCTCAGGGACCAGGCTTTGG
TAATAGAGGATCTGAGAAATGTGACTCCACCCAAGGTCTCCTTGTTTGAG
CCATCAAAAGCAGAGATTGCAAACAAACAAAAGGCTACCCTCGTGTGCT
TGGCCAGGGGCTTCTTCCCTGACCACGTGGAGCTGAGCTGGTGGGTGAA
TGGCAAGGAGGTCCACAGTGGGGTCAGCACGGACCCTCAGGCCTACAA
GGAGAGCAATTATAGCTACTGCCTGAGCAGCCGCCTGAGGGTCTCTGCT
ACCTTCTGGCACAATCCTCGAAACCACTTCCGCTGCCAAGTGCAGTTCC

ATGGGCTTTCAGAGGAGGACAAGTGGCCAGAGGGCTCACCCAAACCTGT
CACACAGAACATCAGTGCAGAGGCCTGGGGCCGAGCAGACTGTGGAAT
CACTTCAGCATCCTATCATCAGGGGGTTCTGTCTGCAACCATCCTCTATG
AGATCCTACTGGGGAAGGCCACCCTATATGCTGTGCTGGTCAGTGGCCT
AGTGCTGATGGCCATGGTCAAGAAAAAAAATTCCTGA

### (4) Inhibition of TCR recognition by antibody

The 2E5 reporter T cells (2 × 10⁴ cells) and the IgG antibody (1 mg/ml) acquired in the above item (2) were added to the 293 T cells pulsed with the PLP peptide obtained in the item (1) in Example 6, and the 293 T cells were cultured at 37°C for one day. The cultured cells were collected, and the 2E5 reporter T cells were stained with an APC-labeled anti-CD45 antibody (clone name: 30-F11, eBioscience). Then, the stained 2E5 reporter cells were analyzed by flow cytometry so as to determine the proportion of the GFP-positive cells in the CD45-positive cells. As Control 1, the proportion of the GFP-positive cells in the CD45-positive cells was analyzed in the same manner, except that the above-described medium was added instead of the IgG antibody. As Control 2, the proportion of the GFP-positive cells in the CD45-positive cells was analyzed in the same manner, except that IgG antibody purified from serums derived from mice immunized without adding the PLP peptide 136-151 (CFA-immunized mice) was used.

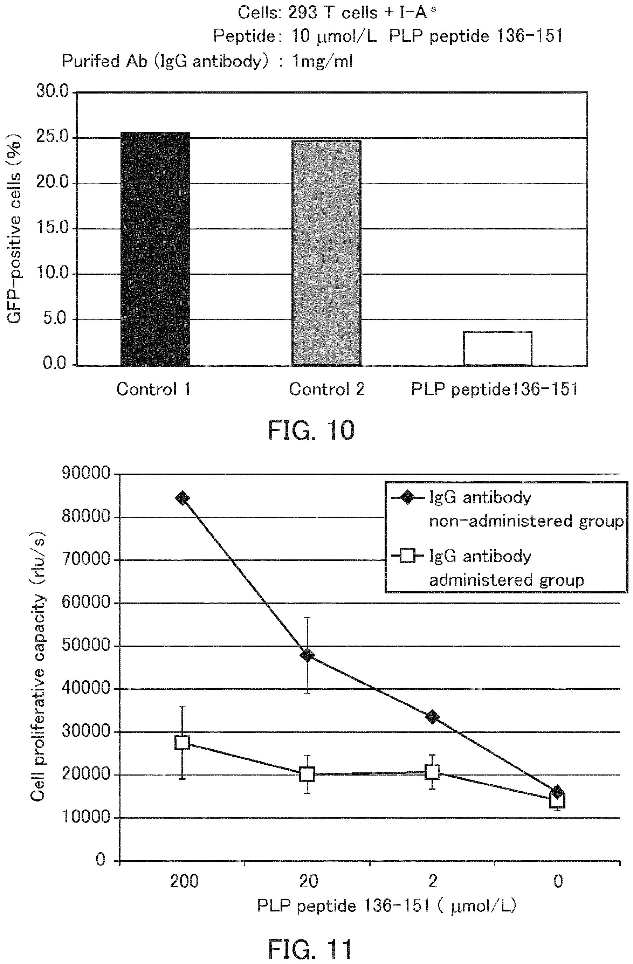

The results obtained are shown in FIG. 10. FIG. 10 is a graph showing the proportion of the GFP-positive cells in the CD45-positive cells. In FIG. 10, the horizontal axis indicates the type of the sample, and the vertical axis indicates the proportion of the GFP-positive cells. As can be seen from FIG. 10, in Control 2 (the IgG antibody purified from the serums of the CFA-immunized mice was used), the proportion of the GFP-positive cells was roughly equivalent to that in Control 1, and the 2E5 TCRs of the 2E5 reporter cells bound to T-cell epitope-MHC class II molecule complexes so that the 2E5 reporter cells were activated. In contrast, when the IgG antibody purified from the serums of the PLP peptide 136-151-immunized mice was added, the proportion of the GFP-positive cells was considerably lower than those in Controls 1 and 2. That is to say, the IgG antibody purified from the serums of the PLP peptide 136-151-immunized mice inhibited the binding between the 2E5 TCRs of the 2E5 reporter cells and the T-cell epitope-MHC class II molecule complexes, thereby inhibiting the activation of the 2E5 reporter cells. These results demonstrate that an antibody obtained as a result of immunization with an immunizing peptide inhibits the binding of a TCR that recognizes a T-cell epitope-MHC class II molecule complex to an immunizing peptide-MHC class II molecule complex.

### [Example 7]

The present example examined whether an antibody obtained as a result of immunization with an immunizing peptide inhibits the activation of T cells (specific T cells) that recognize immunizing peptide-MHC class II molecule complexes in a living organism.

### (1) Acquisition of IgG antibody

An IgG antibody was purified in the same manner as in the item (2) in Example 6.

### (2) Activation of specific T cells in living organism

30 nmol of the PLP peptide 139-151 was mixed with CFA, and mice (SJL/J strain) were immunized subcutaneously with the resultant mixture. On Day 0 and Day 2 after the immunization, 2 mg of the IgG antibody acquired in the item (1) in Example 7 was administered intraperitoneally to the mice. On Day 11 after the immunization, CD4-positive cells were purified from the lymph nodes of the mice using an anti-CD4 antibody (clone name: GK1.5, eBioscience) and a cell sorter (Sony cell sorter SH800Z, Sony).

The spleen collected from each of non-immunized mice (SJL/J strain) was homogenized to prepare a single cell suspension. Thereafter, by lysing the erythrocytes with an ACK lysis buffer, spleen cells were prepared. Then, the CD4-positive cells (1 × 10⁵ cells) and the spleen cells (4 × 10⁵ cells) were mixed together, and the PLP peptide 136-151 was added thereto at a predetermined concentration (0, 2, 20, or 200 µmol/l). The cells were cultured at 37°C for 4 days, thereby stimulating the CD4-positive cells to induce proliferation. The culture was performed using an RPMI medium. Then, the cultured cells were measured using a cell proliferation measurement kit (Cell proliferation ELISA kit, Roche) (IgG antibody administered group). As a control, the measurement was performed in the same manner, except that CD4-positive cell purified from mice to which the IgG antibody had not been administered were used (IgG antibody non-administered group).

The results obtained are shown in FIG. 11. FIG. 11 is a graph showing the results of measuring the cell proliferation. In FIG. 11, the horizontal axis indicates the concentration of the PLP peptide 136-151, and the vertical axis indicates the cell proliferative capacity (rlu/s). In FIG. 11, open squares (□) indicate the results obtained when the CD4-positive cells derived from the mice to which the IgG antibody had been administered were used, and the filled rhombuses (◆) indicate the results obtained regarding the control. As can be seen from FIG. 11, when the PLP peptide 136-151 was not added, i.e., when the CD4-positive cells were not stimulated, there was no difference in the proliferative capacity of the CD4-positive cells between the IgG antibody administered group and the IgG antibody non-administered group. In contrast, when the concentration of the PLP peptide 136-151 was 2, 20, or 200 µmol/l, the proliferative capacity of the CD4-positive cells in the IgG antibody administered group was much lower than that in the IgG antibody non-administered group. These results demonstrate that an antibody obtained as a result of immunization with an immunizing peptide inhibits the activation of T cells that recognize immunizing peptide-MHC class II molecule complexes in a living organism.

### (3) Evaluation of activation of specific T cells using adoptive transfer

Immunization with the PLP peptide 139-151 and administration of the IgG antibody were performed in the same manner as in the item (2) in Example 7. Then, on Day 12 after the immunization, a single cell suspension was prepared by homogenizing the lymph nodes of the mice, and lymph node cells were prepared. Then, the PLP peptide 139-151 was added to the lymph node cells at a concentration of 50 µg/ml, and the lymph node cells were cultured at 37°C for 4 days. The cultured lymph node cells were collected, and intravenously administered to non-immunized mice (SJL/J strain) at a density of 9 × 10⁶ cells/mouse. Then, on predetermined days (0, 3, 5, 8, 10, 12, 14, 17, 19, 22, 25, 28, 32, and 36 days) after the administration, the clinical score (EAE Score) was determined according to the following autoimmune encephalomyelitis evaluation criteria (IgG antibody administered group). As a control, the clinical score was determined in the same manner, except that lymph node cells prepared from mice to which the IgG antibody had not been administered were used (IgG antibody non-administered group).

### (Autoimmune encephalomyelitis evaluation criteria)

### Score: symptoms

1: flaccid tail (the tail remains drooping down when held by an experimenter)
2: partial hind-limb paralysis (abnormal gait)
3: total hind-limb paralysis
4: hind-limb and hindquarter paralysis
5: hind-limb, hindquarter, and fore-limb paralysis
6: dead

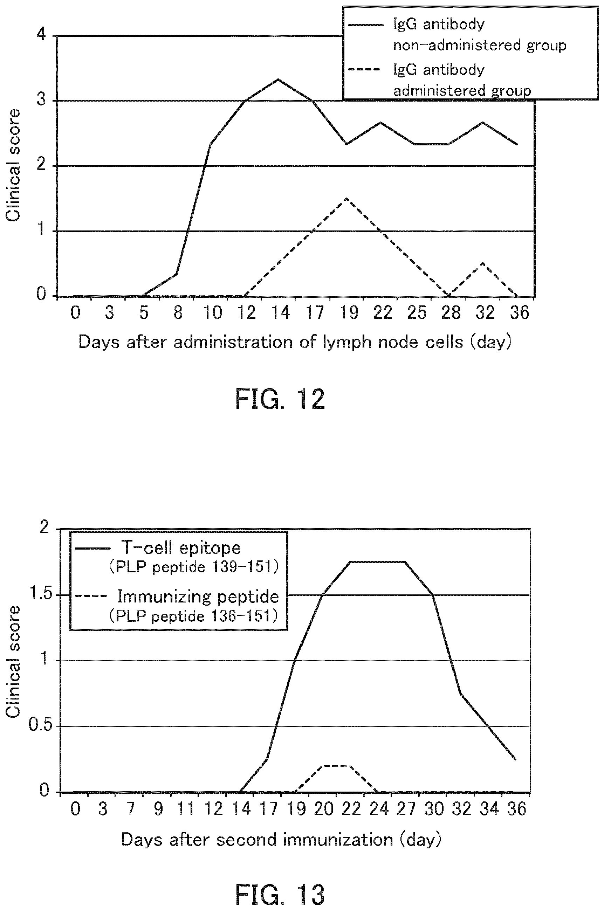

The results obtained are shown in FIG. 12. FIG. 12 is a graph showing the results of the clinical score evaluation. In FIG. 12, the horizontal axis indicates the number of days elapsed after the administration of the lymph node cells, and the vertical axis indicates the clinical score. In FIG. 12, the dashed line indicates the result obtained regarding the IgG antibody administered group, and the solid line indicates the result obtained regarding the IgG antibody non-administered group. As can be seen from FIG. 12, the mice in the IgG antibody administered group developed the autoimmune encephalomyelitis later than the mice in the IgG antibody non-administered group. Besides, even if they had developed the autoimmune encephalomyelitis, they showed significantly reduced symptoms as compared with the mice in the IgG antibody non-administered group. These results demonstrate that an antibody obtained as a result of immunization with an immunizing peptide inhibits the activation of T cells that recognize immunizing peptide-MHC class II molecule complexes in a living organism.

### [Example 8]

The present example examined whether immunization with an immunizing peptide can inhibit the onset and symptoms of experimental autoimmune encephalomyelitis (EAE), which is an autoimmune disease.

30 nmol of the PLP peptide 136-151 was mixed with CFA, and mice (SJL/J strain) were immunized subcutaneously with the resultant mixture. At 8 weeks after the immunization, 50 nmol of the PLP peptide 139-151 was mixed with CFA, and the mice were immunized with the resultant mixture. Further, on Day 0 and Day 2 after the immunization with the PLP peptide 139-151, EAE was induced by intravenously administering 100 ng of a pertussis toxin to the mice. Then, on predetermined days (0, 3, 7, 9, 11, 12, 14, 17, 19, 20, 22, 24, 27, 30, 32, 34, and 36 days) after the immunization with the PLP peptide 139-151, the clinical score (EAE Score) was determined according to the following autoimmune encephalomyelitis evaluation criteria for (the immunizing peptide administered group). As a control, the clinical score was determined in the same manner, except that, at the time of the first immunization, the PLP peptide 139-151 was used instead of the PLP peptide 136-151 (T-cell epitope administered group).

The results obtained are shown in FIG. 13. FIG. 13 is a graph showing the results of the clinical score evaluation. In FIG. 13, the horizontal axis indicates the number of days elapsed after the second immunization, and the vertical axis indicates the clinical score. In FIG. 13, the dashed line indicates the result obtained regarding the immunizing peptide administered group, and the solid line indicates the result obtained regarding the T-cell epitope administered group. As can be seen from FIG. 13, the mice in the immunizing peptide administered group developed the autoimmune encephalomyelitis later than the mice in the T-cell epitope administered group. Besides, even if they had developed the autoimmune encephalomyelitis, they showed drastically reduced symptoms as compared with the mice in the T-cell epitope administered group. These results demonstrate that immunization with an immunizing peptide can inhibit the onset and symptoms of an autoimmune disease.

While the present invention has been described above with reference to illustrative embodiments and examples, the present invention is by no means limited thereto. Various changes and modifications that may become apparent to those skilled in the art may be made in the configuration and specifics of the present invention without departing from the scope of the present invention.

This application claims priority from Japanese Patent Application No. 2015-068043 filed on March 30, 2015. The entire disclosure of this Japanese patent application is incorporated herein by reference.

### Industrial Applicability

The immunizing peptide of the present invention can induce production of an antibody to a complex of the immunizing peptide and an MHC molecule of a living organism. Thus, the present invention is very useful in the field of clinical practice etc., for example.

### [Sequence Listing]

TF14085WO_ST25.txt

### Aspects of the disclosure

1. An immunizing peptide comprising:
   a T-cell epitope of a target protein; and
   an antibody-inducing portion of the target protein,
   wherein the antibody-inducing portion is at least one of the following amino acid residues in an amino acid sequence of the target protein: one or more contiguous amino acid residues immediately upstream of a N-terminal amino acid residue of the T-cell epitope; and one or more contiguous amino acid residues immediately downstream of a C-terminal amino acid residue of the T-cell epitope, and
   when the immunizing peptide is administered to a living organism, the immunizing peptide induces production of an antibody to a complex of the immunizing peptide and an MHC molecule of the living organism.
2. The immunizing peptide according to aspect 1, wherein
   the antibody-inducing portion is at least one of the following amino acid residues in the amino acid sequence of the target protein: three or more contiguous amino acid residues immediately upstream of the N-terminal amino acid residue of the T-cell epitope; and three or more contiguous amino acid residues immediately downstream of the C-terminal amino acid residue of the T-cell epitope.
3. The immunizing peptide according to aspect 1 or 2, wherein
   the produced antibody inhibits binding of a T-cell receptor that recognizes a complex of the T-cell epitope and the MHC molecule of the living organism.
4. The immunizing peptide according to any one of aspects 1 to 3, wherein
   the T-cell epitope is a substituted T-cell epitope configured so that: the substituted T-cell epitope consists of an amino acid sequence obtained by substitution of one or more amino acid residues recognized by a T-cell receptor in the amino acid sequence of the T-cell epitope; and a complex of the substituted amino acid sequence and the MHC molecule is not recognized by the T-cell receptor specific to a complex of the T-cell epitope and the MHC molecule.
5. The immunizing peptide according to any one of aspects 1 to 4, further comprising a B-cell epitope of the target protein, wherein
   the B-cell epitope is the following substituted B-cell epitope (1) or (2):
   (1) a substituted B-cell epitope that consists of an amino acid sequence obtained by deletion, substitution, insertion, and/or addition of one or more amino acid residues in an amino acid sequence of the B-cell epitope and is not recognized by a B-cell receptor specific to the B-cell epitope; and
   (2) a substituted B-cell epitope that consists of an amino acid sequence having less than 100% sequence identity to the amino acid sequence of the B-cell epitope and is not recognized by the B-cell receptor specific to the B-cell epitope.
6. The immunizing peptide according to any one of aspects 1 to 5, wherein
   the MHC molecule is at least one of an MHC class I molecule and an MHC class II molecule.
7. The immunizing peptide according to any one of aspects 1 to 6, wherein
   the target protein is an antigenic protein involved in an immune disease.
8. The immunizing peptide according to aspect 7, wherein
   the immune disease is an allergic disease or an autoimmune disease.
9. The immunizing peptide according to aspect 8, wherein
   the allergic disease is one selected from the group consisting of food allergies, oral allergy syndrome, drug allergies, pollinosis, atopic dermatitis, allergic conjunctivitis, eosinophilic pneumonia, allergic rhinitis, allergic gastroenteritis, contact dermatitis, urticaria, photosensitivity, metal allergies, cat allergies, mite allergies, and asthma.
10. The immunizing peptide according to aspect 8, wherein
   the autoimmune disease is one selected from the group consisting of rheumatoid arthritis, type I diabetes, systemic lupus erythematosus, Basedow's disease, Hashimoto's disease, psoriasis, pemphigus, bullous pemphigoid, scleroderma, dermatomyositis, interstitial pneumonia, pulmonary alveolar proteinosis, ANCA-associated vasculitis, polymyalgia rheumatica, autoimmune hepatitis, celiac disease, polymyositis, Sjogren's syndrome, IgG4-related disease, vasculitic syndrome, mixed connective tissue disease, Guillain-Barre syndrome, myasthenia gravis, chronic gastritis, chronic atrophic gastritis, primary biliary cirrhosis, ulcerative colitis, Crohn's disease, primary sclerosing cholangitis, autoimmune pancreatitis, aortitis syndrome, Goodpasture's syndrome, rapidly progressive glomerulonephritis, membranous nephropathy, IgA nephropathy, minimal change nephrotic syndrome, megaloblastic anemia, autoimmune hemolytic anemia, autoimmune neutropenia, idiopathic thrombocytopenic purpura, primary hypothyroidism, idiopathic Addison's disease, chronic discoid lupus erythematosus, localized scleroderma, herpes gestationis, linear IgA bullous dermatosis, epidermolysis bullosa acquisita, alopecia areata, vilitigo vulgaris, leukoderma acquisitum centrifugum of Sutton/Sutton nevus, Harada disease, autoimmune optic neuropathy, anti-cardiolipin antibody syndrome, autoimmune inner ear disorder, idiopathic azoospermia, habitual abortion, Behcet's disease, ankylosing spondylitis, multiple sclerosis, and narcolepsy.
11. A method for producing an immunizing peptide, the method comprising the step of:
   expressing a polynucleotide encoding the immunizing peptide according to any one of aspects 1 to 10.
12. A pharmaceutical composition for an immune disease, the pharmaceutical composition comprising:
   the immunizing peptide according to any one of aspects 1 to 10.
13. The pharmaceutical composition according to aspect 12, further comprising an adjuvant.
14. A method for treating an immune disease, the method comprising:
   administering to a living organism at least one of the immunizing peptide according to any one of aspects 1 to 10 and the pharmaceutical composition according to aspects 12 or 13.

## Claims

1. A method for inducing production of an antibody, the method comprising:
administering an immunizing peptide to a non-human living organism, wherein the immunizing peptide comprises:
a T-cell epitope of a target protein; and
an antibody-inducing portion of the target protein, wherein the antibody-inducing portion is at least one of the following:
one or more contiguous amino acid residues immediately upstream of a N-terminal amino acid residue of the T-cell epitope in the amino acid sequence of the target protein; and
one or more contiguous amino acid residues immediately downstream of a C-terminal amino acid residue of the T-cell epitope in the amino
acid sequence of the target protein; and
collecting serum from the non-human living organism.

2. The method according to claim 1, further comprising purifying from the serum an antibody that binds to a complex of the immunizing peptide and an MHC molecule of the living organism.

3. The method according to claim 1 or 2, wherein the produced antibody inhibits binding of a T-cell receptor that recognizes a complex of the T-cell epitope and the MHC molecule of the living organism.

4. The method according to any one of claims 1 to 3, wherein the antibody-inducing portion is at least one of the following:
three or more contiguous amino acid residues immediately upstream of the N-terminal amino acid residue of the T-cell epitope in the amino acid sequence of the target protein; and
three or more contiguous amino acid residues immediately downstream of the C-terminal amino acid residue of the T-cell epitope in the amino acid sequence of the target protein.

5. The method according to any one of the preceding claims, wherein the T-cell epitope is a substituted T-cell epitope configured so that: the substituted T-cell epitope consists of an amino acid sequence obtained by substitution of one or more amino acid residues recognized by a T-cell receptor in the amino acid sequence of the T-cell epitope; and a complex of the substituted amino acid sequence and the MHC molecule is not recognized by the T-cell receptor specific to a complex of the T-cell epitope and the MHC molecule.

6. The method according to any one of the preceding claims, wherein the immunizing peptide comprises a B-cell epitope of the target protein, and the B-cell epitope is the following substituted B-cell epitope (1) or (2):
(1) a substituted B-cell epitope that consists of an amino acid sequence obtained by deletion, substitution, insertion, and/or addition of one or more amino acid residues in an amino acid sequence of the B-cell epitope and is not recognized by a B-cell receptor specific to the B-cell epitope; and
(2) a substituted B-cell epitope that consists of an amino acid sequence having less than 100% sequence identity to the amino acid sequence of the B-cell epitope and is not recognized by the B-cell receptor specific to the B-cell epitope.

7. The method according to any one of the preceding claims, wherein the MHC molecule is at least one of an MHC class I molecule and an MHC class II molecule.

8. The method according to any one of the preceding claims, wherein the target protein is an antigenic protein involved in an immune disease.

9. The method according to claim 8, wherein the immune disease is an allergic disease or an autoimmune disease.

10. The method according to claim 9, wherein the allergic disease is one selected from the group consisting of food allergies, oral allergy syndrome, drug allergies, pollinosis, atopic dermatitis, allergic conjunctivitis, eosinophilic pneumonia, allergic rhinitis, allergic gastroenteritis, contact dermatitis, urticaria, photosensitivity, metal allergies, cat allergies, mite allergies, and asthma.

11. The method according to claim 9, wherein the autoimmune disease is one selected from the group consisting of rheumatoid arthritis, type I diabetes, systemic lupus erythematosus, Basedow's disease, Hashimoto's disease, psoriasis, pemphigus, bullous pemphigoid, scleroderma, dermatomyositis, interstitial pneumonia, pulmonary alveolar proteinosis, ANCA-associated vasculitis, polymyalgia rheumatica, autoimmune hepatitis, celiac disease, polymyositis, Sjogren's syndrome, IgG4-related disease, vasculitic syndrome, mixed connective tissue disease, Guillain-Barre syndrome, myasthenia gravis, chronic gastritis, chronic atrophic gastritis, primary biliary cirrhosis, ulcerative colitis, Crohn's disease, primary sclerosing cholangitis, autoimmune pancreatitis, aortitis syndrome, Goodpasture's syndrome, rapidly progressive glomerulonephritis, membranous nephropathy, IgA nephropathy, minimal change nephrotic syndrome, megaloblastic anemia, autoimmune hemolytic anemia, autoimmune neutropenia, idiopathic thrombocytopenic purpura, primary hypothyroidism, idiopathic Addison's disease, chronic discoid lupus erythematosus, localized scleroderma, herpes gestationis, linear IgA bullous dermatosis, epidermolysis bullosa acquisita, alopecia areata, vilitigo vulgaris, leukoderma acquisitum centrifugum of Sutton/Sutton nevus, Harada disease, autoimmune optic neuropathy, anti-cardiolipin antibody syndrome, autoimmune inner ear disorder, idiopathic azoospermia, habitual abortion, Behcet's disease, ankylosing spondylitis, multiple sclerosis, and narcolepsy.

12. An antibody that binds to a complex of an immunizing peptide and an MHC molecule of a living organism;
wherein the immunizing peptide comprises:
a T-cell epitope of a target protein; and
an antibody-inducing portion of the target protein,
wherein the antibody-inducing portion is at least one of the following: one or more contiguous amino acid residues immediately upstream of a N-terminal amino acid residue of the T-cell epitope in the amino acid sequence of the target protein; and one or more contiguous amino acid residues immediately downstream of a C-terminal amino acid residue of the T-cell epitope in the amino acid sequence of the target protein.

13. The antibody according to claim 12, wherein the immunizing peptide is as defined in any one of claims 4 to 11.

14. An antibody produced by the method of any one of claims 1 to 11.

15. The antibody according to any one of claims 12 to 14, wherein the antibody inhibits binding of a T-cell receptor that recognizes a complex of the T-cell epitope and the MHC molecule of the living organism.

16. The antibody according to any one of claims 12 to 15 for use in a method of treating an immune disease.
